# FASCICULE DE BREVET EUROPEEN

(11) **EP 3 878 971 B1**
(45) Date de publication et mention de la délivrance du brevet: **04.12.2024**
(21) Numéro de dépôt: 21160980.5
(22) Date de dépôt: 05.03.2021
(51) Int. Cl.: C12Q 1/6804, C12Q 1/6851

(54) **PROCÉDÉ POUR DÉTECTER ET ÉVENTUELLEMENT QUANTIFIER UN ANALYTE AVEC UN OLIGONUCLÉOTIDE À DOUBLE STRUCTURE TIGE-BOUCLE**
VERFAHREN ZUM NACHWEIS UND GEGEBENENFALLS ZUR QUANTIFIZIERUNG EINES ANALYTEN MIT EINEM OLIGONUKLEOTID MIT DOPPELTER HAARNADELSTRUKTUR
METHOD FOR DETECTING AND POSSIBLY QUANTIFYING AN ANALYTE WITH AN OLIGONUCLEOTIDE WITH DOUBLE STEM-LOOP STRUCTURE

(30) Priorité: 10.03.2020 FR 2002366; 29.09.2020 FR 2009907
(43) Date de publication de la demande: 15.09.2021
(73) Titulaire: Commissariat à l'Energie Atomique et aux Energies Alternatives, 75015 Paris (FR); Centre National de la Recherche Scientifique, 75794 Paris Cedex 16 (FR); Université Grenoble Alpes, 38400 Saint-Martin-d'Hères (FR)
(72) Inventeur: SAVONNET, Maud, 38054 GRENOBLE Cedex 09 (FR); BUHOT, Arnaud, 38054 GRENOBLE Cedex 09 (FR); CUBIZOLLES, Myriam-Laure, 38054 GRENOBLE Cedex 09 (FR); ROUPIOZ, Yoann, 38570 GONCELIN (FR)
(74) Mandataire: Brevalex

(56) Documents cités:
- EP-A1- 0 820 508
- WO-A1-2017/035090
- CN-A- 106 148 549
- MOHAMMAD POURHASSAN-MOGHADDAM ET AL: "Protein detection through different platforms of immuno-loop-mediated isothermal amplification", NANOSCALE RESEARCH LETTERS, vol. 8, no. 1, 18 November 2013 (2013-11-18), pages 485, XP055743482, ISSN: 1556-276X, DOI: 10.1186/1556-276X-8-485
- JIAWEI YE ET AL: "Research advances in the detection of miRNA", JOURNAL OF PHARMACEUTICAL ANALYSIS, vol. 9, no. 4, 25 May 2019 (2019-05-25), pages 217 - 226, XP055743651, ISSN: 2095-1779, DOI: 10.1016/j.jpha.2019.05.004
- CHEN WEIWEI ET AL: "DNA nanomachine-assisted magnetic bead based target recycling and isothermal amplification for sensitive fluorescence determination of interferon-[gamma]", MIKROCHIMICA ACTA, SPRINGER VERLAG, VIENNA, AT, vol. 184, no. 12, 31 October 2017 (2017-10-31), pages 4869 - 4877, XP036354904, ISSN: 0026-3672, [retrieved on 20171031], DOI: 10.1007/S00604-017-2511-X

## Description

### DOMAINE TECHNIQUE

La présente invention concerne le domaine des outils et procédés de détection de molécules d'intérêt.

Plus particulièrement, la présente invention propose un procédé pour détecter et éventuellement quantifier un analyte d'intérêt dans un échantillon liquide mettant en oeuvre un oligonucléotide à double structure tige-boucle et une amplification LAMP (pour « Loop-mediated isothermal AMPlification ») à deux amorces.

### ÉTAT DE LA TECHNIQUE ANTÉRIEURE

De nombreux travaux et études dans des domaines englobant notamment la recherche académique ; la recherche et les analyses, cliniques et diagnostiques, avec des essais réalisés sur des prélèvements de sang ou des biopsies cellulaires ou tissulaires ; la surveillance environnementale; la surveillance civile ; la sécurité alimentaire et notamment le contrôle qualité ; les analyses criminelles sur traces... impliquent la détection de molécules d'intérêt dans un échantillon et notamment dans un échantillon liquide.

Plusieurs techniques ont été mises au point à cet effet.

Une première technique immuno-enzymatique de détection intitulée méthode ELISA (pour « Enzyme-Linked ImmunoSorbent Assay ») a été développée au début des années 1970. Dans cette technique, un sandwich entre deux anticorps spécifiques et la cible peut être réalisé. Le premier anticorps est utilisé pour capturer la cible, par exemple, au fond d'un puits, alors que le deuxième anticorps sert à l'amplification enzymatique, directement ou via un anticorps secondaire couplé à une enzyme comme la HRP (pour « Horse Radish Peroxidase »). La méthode ELISA est spécifique d'un anticorps et permet une quantification de la cible au moyen d'une gamme de quantifications réalisée au préalable ou simultanément. Cette méthode est encore actuellement utilisée et de nombreuses variantes existent. En effet, un aptamère qui est une séquence oligonucléotidique sélectionnée spécifiquement pour son interaction avec un analyte ou toute autre séquence oligonucléotidique ayant une affinité spécifique envers un analyte peut être utilisé(e) à la place d'un anticorps. Les aptamères constituent une bonne alternative dans la mesure où ils peuvent détecter de petites molécules et où ils sont très spécifiques d'une cible, beaucoup plus faciles à produire et à manipuler, plus stables et moins coûteux.

Cependant, lorsque la cible à détecter est présente en très faibles concentrations dans l'échantillon, la technique ELISA avec anticorps ou aptamères s'avère inefficace. Pour résoudre ce problème, l'amplification d'une sonde ADN est généralement nécessaire.

C'est pourquoi, d'autres méthodes de détection ont été développées mettant en oeuvre une amplification en chaîne par polymérase (ou PCR pour « Polymerase Chain Reaction »). A titre d'exemples particuliers de telles méthodes, on peut citer l'immuno-PCR et l'aptaméro-PCR.

Dans l'immuno-PCR, l'amplification enzymatique de la technique ELISA est remplacée par une amplification d'ADN. Le second anticorps est alors couplé à une séquence ADN qui est exponentiellement amplifiée par PCR. Cette méthode est très spécifique et quantitative mais elle nécessite toujours la présence de deux anticorps spécifiques de la cible et notamment le développement d'un anticorps couplé à un ADN, ce qui rend cette méthode compliquée et coûteuse.

L'aptaméro-PCR, quant à elle, permet de remplacer l'anticorps secondaire par un oligonucléotide contenant une séquence d'aptamère spécifique de la cible à détecter. La détection est obtenue par l'amplification directe de cette sonde par PCR. Cette méthode permet d'atteindre une limite de détection de quelques picomolaires. Cependant, l'immuno-PCR et l'aptaméro-PCR présentent, toutes deux, les inconvénients de la PCR à savoir la nécessité de cycles de températures longs et comportant différentes températures.

Enfin, une autre méthode d'amplification a été développée pour remplacer la PCR dans le cadre de l'amplification de doubles brins d'ADN. Cette méthode appelée LAMP (pour « Loop-mediated isothermal AMPlification » et brevetée [1] présente l'avantage majeur d'être isotherme, l'amplification étant réalisée à une température constante typiquement comprise entre 60°C et 65°C.

Ainsi, une méthode de détection de protéines avec amplification LAMP a été décrite par Pourhassan-Moghaddam *et al,* 2013 [2]. Elle met en oeuvre, d'une part, un anticorps ou un aptamère fixé à une surface pour capturer la cible et, d'autre part, un oligonucléotide contenant une séquence aptamère qui reconnaît la cible. Cet oligonucléotide est, par la suite, amplifié par la méthode LAMP classique avec quatre amorces.

Cependant, le design complexe des quatre amorces indispensables à la méthode d'amplification LAMP rend ce procédé complexe et coûteux à mettre en oeuvre.

Enfin, une méthode de détection de micro-ARNs (mi-ARNs) par une méthode LAMP avec deux amorces a été développée [3,4]. Cette méthode présente l'avantage d'être quantitative. Toutefois, elle ne permet de détecter que de l'ARN à partir duquel est formé un fragment d'ADN complémentaire (ADNc) au moyen d'une réaction de transcription inverse. Deux sondes d'ADN en épingle à cheveux désignées H1 et H2 dans Du *et al,* 2016 [4] sont conçues, la sonde H1 présentant à son extrémité 3' une extension complémentaire à la moitié du fragment d'ADNc et la sonde H2 présentant à son extrémité 5' une extension complémentaire à l'autre moitié du fragment d'ADNc. En présence du fragment d'ADNc, les sondes H1 et H2 s'hybrident sur ce dernier et, si aucun mésappariement n'existe entre les extensions des sondes H1 et H2 et le fragment d'ADNc, une liaison covalente entre les deux sondes d'ADN H1 et H2 peut être formée grâce à une ligase haute-fidélité comme la *Taq* DNA ligase. Il est donc clair que cette méthode de détection de mi-ARNs n'est pas générique puisqu'elle nécessite la préparation de deux sondes spécifiques de chaque mi-ARN à détecter. Par ailleurs, cette méthode implique plusieurs étapes avant la mise en oeuvre de la technique LAMP avec une étape de transcription inverse et une étape de ligation, cette dernière pouvant être complexe avec un rendement possiblement faible et la nécessité d'utiliser une enzyme supplémentaire. Malgré ce, dans leur publication Du *et al,* 2016 [4], les auteurs insistent sur le fait que cette étape est primordiale notamment pour une meilleure discrimination des mutations dans les mi-ARNs (phrase pontant les colonnes de la page 12722).

MOHAMMAD POURHASSAN-MOGHADDAM ET AL (2013-11-18) décrit une méthode de détection de protéines avec amplification LAMP. Elle met en oeuvre, d'une part, un anticorps ou un aptamère fixé à une surface pour capturer la cible et, d'autre part, un oligonucléotide contenant une séquence aptamère qui reconnaît la cible. Cet oligonucléotide est, par la suite, amplifié par la méthode LAMP classique avec un jeu d'amorces internes (Forward Internal primer (FIP), Backward Internal primer (BIP)) et un jeu d'amorces externes (F3 (forward outer primer) and B3 (backward outer primer)). Il existe donc un réel besoin pour une méthode permettant de détecter des molécules d'intérêt de nature variée dans un échantillon qui ne présente pas les inconvénients des méthodes de l'état de la technique. Les inventeurs se sont donc fixé pour but de résoudre un tel problème technique.

### EXPOSÉ DE L'INVENTION

La présente invention permet de résoudre des problèmes techniques et inconvénients précédemment listés. En effet, les inventeurs ont proposé un procédé de détection et éventuellement de quantification d'un analyte mettant en oeuvre un outil oligonucléotidique qui permet de profiter des avantages de l'amplification LAMP à deux amorces, à savoir méthode isotherme et quantifiable ne nécessitant que deux amorces. Ainsi, l'amplification LAMP du procédé de détection selon l'invention est plus rapide, moins complexe à réaliser et moins coûteuse en raison de la réduction du nombre d'amorces. Par ailleurs, le procédé selon la présente invention met en oeuvre au plus un anticorps spécifique de l'analyte à détecter et peut même n'en mettre aucun en oeuvre.

L'outil oligonucléotidique utilisé dans le cadre de la présente invention est un oligonucléotide à double structure tige-boucle. Les expressions et terme « oligonucléotide à double structure tige-boucle », « oligonucléotide à deux structures tige-boucle », « oligonucléotide à structure d'haltère » et « haltère » sont équivalents et utilisables de façon interchangeable dans le cadre de la présente invention.

Les inventeurs ont montré qu'il était possible d'introduire, dans l'une quelconque des portions de cet haltère, une séquence spécifique de l'analyte à détecter et ce, sans que l'amplification LAMP à deux amorces ne soit inhibée (voir point Il de la partie expérimentale ci-après). Il est également possible d'introduire plusieurs séquences spécifiques, identiques ou différentes, de l'analyte à détecter dans une ou plusieurs des portions de cet haltère, moyennant quoi la sensibilité du procédé de détection est notablement améliorée. On peut également mettre en oeuvre un haltère dans lequel sont présentes plusieurs séquences spécifiques de différents analytes à détecter permettant ainsi la détection d'un ou plusieurs de ces analytes dans un liquide d'intérêt. Dans cette forme de mise en oeuvre, la spécificité de la détection, si souhaitée, est assurée par la sonde apte à lier un analyte donné et immobilisée à la surface d'un support solide.

L'oligonucléotide à double structure tige-boucle mis en oeuvre dans le cadre de la présente invention se distingue clairement des oligonucléotides à double structure tige-boucle de l'art antérieur.

En effet, dans un procédé de détection tel que décrit par Pourhassan-Moghaddam *et al,* 2013 [2], un tel oligonucléotide est un des produits générés durant une amplification LAMP à quatre amorces. Au contraire, dans le cadre de la présente invention, cet oligonucléotide est un réactif synthétique présent avant la mise en contact de la surface sur laquelle sont éventuellement immobilisés l'analyte à détecter et les différents éléments nécessaires à une amplification LAMP comme des amorces et l'enzyme catalysant cette amplification.

De même, l'oligonucléotide à double structure tige-boucle mis en oeuvre dans le cadre de la présente invention se distingue clairement de l'oligonucléotide décrit dans [3,4]. Ce dernier est obligatoirement produit en présence de l'analyte à détecter puisque l'ADNc obtenu à partir de cet analyte sert de matrice lors de l'étape de ligation aboutissant à un oligonucléotide à double structure tige-boucle. Dans le cadre de la présente invention, l'oligonucléotide est synthétisé en l'absence de l'analyte à détecter. Par ailleurs, dans l'oligonucléotide décrit dans [3,4], la séquence comprise entre les tiges des deux structures tige-boucle ne correspond pas, à proprement parler, à une séquence de reconnaissance spécifique du mi-ARN à détecter puisque du fait de l'intermédiaire que constitue l'ADNc, cette séquence est la version ADN de la séquence du mi-ARN et de fait ne constitue pas une séquence de reconnaissance spécifique du mi-ARN à détecter apte à se lier spécifiquement au mi-ARN. Enfin, alors que la portion comprise entre les tiges des deux structures tige-boucle est essentielle au procédé décrit dans [3,4], les inventeurs de la présente ont montré qu'il était possible d'utiliser des oligonucléotides à double structure tige-boucle ne présentant pas une telle portion (voir point III de la partie expérimentale ci-après).

Ainsi, la présente invention concerne un procédé pour détecter et éventuellement quantifier un analyte éventuellement présent dans un échantillon liquide, comprenant les étapes suivantes :
i) mettre en contact ledit échantillon liquide avec la surface d'un support solide comprenant au moins une zone active sur laquelle au moins une sonde apte à lier ledit analyte est immobilisée ;
ii) mettre en contact ladite surface avec une solution contenant au moins un oligonucléotide à double structure tige-boucle, capable de se lier audit analyte, ledit oligonucléotide ayant été synthétisé préalablement à ladite mise en contact ;
iii) laver ladite surface ;
iv) mettre en contact ladite surface avec deux amorces d'amplification isotherme médiée par des boucles dans des conditions permettant l'amplification dudit oligonucléotide ;
v) détecter et éventuellement quantifier le produit de l'amplification dudit oligonucléotide (moyennant quoi ledit analyte est détecté et éventuellement quantifié).

L'échantillon liquide mis en oeuvre dans le cadre de la présente invention est un liquide susceptible de contenir l'analyte à détecter et éventuellement à quantifier. Il peut être de nature et d'origine très variées.

Cet échantillon liquide est avantageusement choisi dans le groupe constitué par un fluide biologique ; un fluide végétal tel que sève, nectar et exsudat racinaire ; un prélèvement dans un milieu de culture ou dans un réacteur de culture biologique comme une culture cellulaire d'eucaryotes supérieurs, de levures, de champignons ou d'algues; un liquide obtenu à partir d'une (ou plusieurs) cellule(s) animale(s) ou végétale(s) ; un liquide obtenu à partir d'un tissu animal ou végétal ; un prélèvement dans une matrice alimentaire ; un prélèvement dans un réacteur chimique ; de l'eau de ville, de rivière, de mer, de piscines, de tours aéro-réfrigérées ou d'origine souterraine; un prélèvement à partir d'un effluent industriel liquide; de l'eau usée provenant notamment d'élevages intensifs ou d'industries du domaine chimique, pharmaceutique ou cosmétique ; un prélèvement provenant d'une filtration d'air ou d'un revêtement ; un prélèvement sur un objet tel qu'un fragment de tissu, un habit, une semelle, une chaussure, un outil ou une arme ; un produit pharmaceutique ; un produit cosmétique ; un parfum ; un échantillon de terre ou un de leurs mélanges.

Dans le cadre de la présente invention, on entend par « prélèvement » tout type de collecte d'échantillons, par exemple, par contact, raclage, forage, découpage, poinçonnage, broyage, lavage, rinçage, aspiration ou pompage.

Le fluide biologique est avantageusement choisi dans le groupe constitué par le sang tel que du sang entier ou du sang entier anti-coagulé, le sérum sanguin, le plasma sanguin, la lymphe, la salive, le crachat, les larmes, la sueur, le sperme, l'urine, les selles, le lait, le liquide céphalo-rachidien, le liquide interstitiel, un fluide isolé de moelle osseuse, un mucus ou fluide du tractus respiratoire, intestinal ou génito-urinaire, des extraits cellulaires, des extraits de tissus et des extraits d'organes. Ainsi, le fluide biologique peut être tout fluide naturellement sécrété ou excrété d'un corps humain ou animal ou tout fluide récupéré, à partir d'un corps humain ou animal, par toute technique connue de l'homme du métier telle qu'une extraction, un prélèvement ou un lavage. Les étapes de récupération et d'isolement de ces différents fluides à partir du corps humain ou animal sont réalisées préalablement à la mise en oeuvre du procédé selon l'invention.

De même, si un des prélèvements envisagés ne permet pas de mettre en oeuvre le procédé de l'invention, par exemple du fait de sa nature gazeuse ou solide, de sa concentration ou des éléments qu'il contient tels que résidus solides, déchets, suspension ou molécules interférentes, le procédé de l'invention comprend en outre une étape préalable de préparation de l'échantillon liquide avec éventuellement une mise en solution du prélèvement par les techniques connues de l'homme du métier telles que filtration, précipitation, dilution, distillation, mélange, concentration, lyse, etc.

Par ailleurs, il est possible d'ajouter, à l'échantillon liquide, des quantités connues d'analyte à détecter. On parle alors d'échantillon liquide dopé par l'analyte, ce dopage permettant d'avoir un contrôle positif notamment lorsque l'échantillon liquide est complexe et éventuellement d'obtenir une quantification de l'analyte à détecter initialement contenu dans l'échantillon liquide.

L'analyte à détecter et éventuellement à quantifier dans l'échantillon liquide peut être choisi dans le groupe constitué par une molécule d'intérêt environnemental tel qu'un pesticide ; une molécule d'intérêt biologique ; une molécule d'intérêt pharmacologique ; une toxine ; un glucide tel que du glucose ; un lipide tel que du cholestérol ; un peptide ; un antigène ; un épitope ; une protéine ; une glycoprotéine ; une enzyme ; un substrat enzymatique ; un récepteur nucléaire ou membranaire ; un agoniste ou un antagoniste d'un récepteur nucléaire ou membranaire ; une hormone ; un anticorps polyclonal ou monoclonal ; un fragment d'anticorps tel qu'un fragment Fab, F(ab')₂, Fv ou un domaine hypervariable (ou CDR pour « Complementarity Determining Region ») ; une molécule nucléotidique ; des ions tels que des ions mercure ou des ions plomb ; une cellule eucaryote ; une cellule procaryote et un virus.

L'expression « molécule nucléotidique » utilisée dans la présente est équivalente aux termes et expressions suivants : « acide nucléique », « polynucléotide », « séquence nucléotidique », « séquence polynucléotidique ». Par « molécule nucléotidique », on entend, dans le cadre de la présente invention, un chromosome ; un gène; un polynucléotide régulateur; un ADN, simple-brin ou double-brin, génomique, chromosomique, chloroplastique, plasmidique, mitochondrial, recombinant ou complémentaire ; un ARN total ; un ARN messager; un ARN ribosomal (ou ribozyme) ; un ARN de transfert ; un micro-ARN ; une séquence faisant office d'aptamère ; une portion ou un fragment de ceux-ci.

A noter que, dans certaines formes de mise en oeuvre, l'analyte à détecter et éventuellement à quantifier dans l'échantillon liquide peut être défini comme une petite molécule i.e. une molécule dont le poids moléculaire est inférieur ou égal à 10000 daltons et notamment inférieur ou égal à 8000 daltons. Cette petite molécule peut appartenir à l'un quelconque des éléments des listes d'analytes ci-dessus.

L'étape i) du procédé selon la présente invention met en oeuvre une sonde apte à lier ledit analyte immobilisée sur la surface d'un support solide. La sonde utilisée pour fonctionnaliser une zone active de la surface du support solide est toute molécule capable de former avec l'analyte à détecter une paire de liaison, la sonde et l'analyte correspondant aux deux partenaires de cette paire de liaison. Les liaisons mises en oeuvre dans la liaison analyte-sonde sont avantageusement soit des liaisons non covalentes et de faible énergie telles que des liaisons hydrogène ou des liaisons de Van der Waals, soit des liaisons de forte énergie de type liaisons covalentes.

La sonde utilisée est donc dépendante de l'analyte à détecter. En fonction de cet analyte, l'homme du métier saura, sans effort inventif, choisir la sonde la plus adaptée. Elle peut être choisie dans le groupe constitué par un glucide ; un peptide ; un antigène ; un épitope ; une protéine ; une glycoprotéine ; une enzyme ; un substrat enzymatique ; un récepteur membranaire ou nucléaire ; un agoniste ou un antagoniste d'un récepteur membranaire ou nucléaire ; une toxine ; un anticorps polyclonal ou monoclonal ; un fragment d'anticorps tel qu'un fragment Fab, F(ab')₂, Fv ou un domaine hypervariable ; une molécule nucléotidique telle que précédemment définie ; un acide nucléique peptidique et un aptamère.

Tout support solide permettant de mettre en oeuvre la présente invention est utilisable. Il peut s'agir, par exemple, d'un support de biopuce tel que ceux classiquement utilisés en silicium, en verre, en métal, en polymère ou en plastique. Ce support solide peut également consister en des particules et notamment des particules de silice, des particules polymériques et/ou des particules magnétisables.

La fonctionnalisation de la zone active de la surface du support solide par une sonde telle que précédemment définie peut être effectuée par toute technique adaptée permettant la fixation d'un composé sur un support solide. On peut en particulier envisager une simple adsorption, des liaisons ioniques, des liaisons hydrogène, des interactions électrostatiques, des interactions hydrophobes, des liaisons de Van der Waals ou un greffage covalent.

Dans une forme de mise en oeuvre particulière de la présente invention, la surface du support solide présente des groupements fonctionnels grâce auxquels la (ou les) sonde(s) est(sont) capable(s) de s'immobiliser. De façon avantageuse, ces groupements fonctionnels sont choisis parmi les groupements carboxyliques, des entités radicalaires, les fonctions alcools, amines, amides, époxy ou thiols. Ces groupements sont portés, de façon intrinsèque ou par fonctionnalisation, par la zone active de la surface du support solide. La (ou les) sonde(s) peuvent également présenter de tels groupements fonctionnels, de façon intrinsèque ou par fonctionnalisation.

Dans une première variante de la présente invention, la (ou les) sonde(s) peu(ven)t être immobilisée(s) de façon directe au niveau de la surface fonctionnalisée ou non. Un support solide « coaté » avec une protéine telle que la streptavidine est un exemple d'immobilisation directe, dans lequel la sonde doit être fonctionnalisée par une biotine.

Dans une seconde variante de la présente invention, la (ou les) sonde(s) peu(ven)t être immobilisée(s) de façon indirecte au niveau de la surface fonctionnalisée ou non. Cette immobilisation indirecte fait intervenir un bras espaceur (ou agent de jonction) lié, d'une part, à la surface et, d'autre part, à une sonde. Un tel bras espaceur est notamment utilisé pour améliorer l'accessibilité à la sonde. A titre d'exemples illustratifs, on peut citer les réactifs silanes mis en oeuvre pour le greffage de sonde(s) sur du verre, la complexation de produits thiolés sur des surfaces d'or et l'immobilisation de sonde(s) dans des matrices polymères. De plus, lorsque la (ou les) sonde(s) est(sont) une (ou des) molécule(s) nucléotidique(s) telle(s) que précédemment définie(s), un tel bras espaceur peut se présenter sous forme d'une séquence comprenant plusieurs bases thymine et notamment 10 bases thymine.

Les liaisons mises en oeuvre au cours d'une immobilisation directe ou indirecte peuvent être toutes liaisons connues de l'homme du métier et notamment des liaisons covalentes, des liaisons ioniques, des liaisons hydrogène, des interactions électrostatiques, des interactions hydrophobes, des liaisons de Van der Waals ou une adsorption.

Avantageusement, une étape de blocage de la surface peut être mise en oeuvre préalablement à la fixation de la sonde et/ou suite à cette fixation. La solution de blocage mise en oeuvre lors de cette étape de blocage peut comporter un ou plusieurs des composants suivants : albumine comme de la BSA (pour « Bovine Serum Albumin »), ADN génomique comme de l'ADN simple brin et notamment de l'ADN simple brin de sperme de saumon, gélatine, caséine, protéine de lait, sérum, polyéthylène glycol et polymères du type polyvinylpyrrolidone. Un exemple particulier de solution de blocage est une solution contenant 5% de BSA et 20 µg/mL d'ADN simple brin de sperme de saumon.

L'étape i) du procédé selon l'invention consiste à mettre en contact l'échantillon liquide tel que précédemment défini avec la surface d'un support solide fonctionnalisée par une ou plusieurs sonde(s) apte(s) à lier l'analyte à détecter moyennant quoi, si l'échantillon liquide contient cet analyte, ce dernier est capturé au niveau de la ou des sonde(s).

Typiquement, l'étape i) de mise en contact peut durerentre 1 min et 24 h, entre 2 min et 12 h, entre 5 min et 6 h, entre 10 min et 3 h, entre 15 min et 2 h, entre 20 min et 1 h et, notamment, de l'ordre de 30 min (i.e. 30 min ± 5 min). Par ailleurs, l'étape i) de mise en contact peut être réalisée à une température comprise entre 4°C et 100°C, notamment entre 10°C et 60°C, en particulier entre 15 et 45°C et, plus particulièrement, à température ambiante (i.e. 23°C ± 5°C) ou à température physiologique (i.e. 37°C ± 5°C).

Suite à l'étape i) et préalablement à l'étape ii) du procédé selon la présente invention, la surface du support solide peut être lavée de façon à éliminer les éléments présents dans l'échantillon liquide et qui n'ont pas été capturés par la ou les sonde(s). Toutefois, cette étape de lavage additionnelle est optionnelle puisque ces éléments pourront être éliminés par la suite et notamment lors de l'étape iii) de lavage.

Lorsque cette étape optionnelle de lavage est mise en oeuvre, la surface du support solide est soumise à au moins un rinçage de façon à éliminer toute trace de l'échantillon liquide. Ce rinçage est typiquement effectué avec une solution aqueuse de rinçage conservant l'interaction sonde/analyte ou liant irréversiblement l'analyte et la sonde. Cette solution peut également permettre d'éliminer les interactions non spécifiques. Elle peut comporter un ou plusieurs des composants suivants : un tampon comme un tampon Tris, phosphate, acétate ou borate ; des sels comme KCI, NaCl, (NH₄)₂SO₄, MgCl₂ ou CaCl₂ ; des détergents ou agents tensioactifs comme Tween^{®}, Triton^{®} ou dodécylsulfate de sodium (ou SDS) ; des agents dénaturants comme formamide ou diméthylsulfoxyde ; un solvant organique comme éthanol, méthanol ou acétonitrile ; et des agents pontants comme formaldéhyde ou glutaraldéhyde. Ce rinçage peut être répété deux fois, trois fois, cinq fois, 10 fois et voire 50 fois en utilisant, à chaque rinçage, une solution de rinçage identique ou différente. Typiquement, le rinçage est répété trois fois avec une solution comprenant du tampon phosphate salin (ou PBS) et 0,1% de Tween^{®}.

De plus, lorsque cette étape optionnelle de lavage est mise en oeuvre, elle est réalisée à une température comprise entre 4°C et 100°C, notamment entre 10°C et 60°C, en particulier entre 15°C et 45°C et, plus particulièrement, à température ambiante ou à température physiologique.

L'étape ii) du procédé selon la présente invention consiste à mettre en contact la surface du support solide sur laquelle l'analyte à détecter est éventuellement retenu via la ou les sonde(s) fonctionnalisant cette surface avec une solution contenant au moins un oligonucléotide à double structure tige-boucle, capable de se lier audit analyte, moyennant quoi, si cet analyte est présent à la surface du support solide, l'oligonucléotide se lie à ce dernier. En d'autres termes, l'oligonucléotide à double structure tige-boucle est capable de former avec l'analyte à détecter une paire de liaison, l'oligonucléotide et l'analyte correspondant aux deux partenaires de cette paire de liaison. Les liaisons mises en oeuvre dans la liaison oligonucléotide-analyte sont avantageusement des liaisons non covalentes et de faible énergie telles que des liaisons hydrogène ou des liaisons de Van der Waals.

Comme précédemment expliqué, l'oligonucléotide a été synthétisé préalablement à la mise en contact de l'étape ii). En d'autres termes, cette synthèse n'est effectuée ni en présence de la surface du support solide fonctionnalisée par la ou les sonde(s) telle(s) que précédemment définie(s), ni en présence de l'analyte à détecter. Typiquement, la synthèse de cet oligonucléotide à double structure tige-boucle ne fait pas intervenir deux des amorces habituellement mises en oeuvre dans une amplification LAMP à quatre amorces. Avantageusement, cette synthèse est une synthèse chimique classiquement utilisée pour préparer des oligonucléotides.

L'oligonucléotide à double structure tige-boucle mis en oeuvre lors de l'étape ii) répond à la formule (I) ou (II) ci-après

5'-F1c-F2-F0'-F1-Int-B1c-B0'-B2c-B1-3' (I)

5'-F1c-F2-F0'-F1-B1c-B0'-B2c-B1-3' (II)

dans lesquelles
la portion F1c présente une séquence nucléotidique complémentaire à la séquence nucléotidique de la portion F1 moyennant quoi la portion F1c et la portion F1 s'hybrident pour former la tige de la première structure tige-boucle,
la portion F2-F0' forme la boucle de la première structure tige-boucle, désignée ci-après boucle F0,
la portion B1 présente une séquence nucléotidique complémentaire à la séquence nucléotidique de la portion B1c moyennant quoi la portion B1 et la portion B1c s'hybrident pour former la tige de la seconde structure tige-boucle,
la portion B0'-B2c forme la boucle de la seconde structure tige-boucle, désignée ci-après boucle B0, et
la portion Int sépare la portion F1 et la portion B1c et comprend au moins un nucléotide.

Dans le cadre de la présente invention, deux séquences nucléotidiques sont complémentaires l'une de l'autre, lorsqu'un nombre suffisant de nucléotides de la première séquence nucléotidique peut se lier, au moyen de liaisons hydrogène, avec les nucléotides correspondants de la deuxième séquence nucléotidique de telle sorte que l'appariement entre les deux séquences nucléotidiques peut se produire. La « complémentarité », telle qu'utilisée ici, se réfère à la capacité d'appariement entre les nucléotides d'une première séquence nucléotidique et d'une seconde séquence nucléotidique. Des nucléotides non complémentaires entre deux séquences nucléotidiques peuvent être tolérés à condition que les deux séquences nucléotidiques restent capables de s'hybrider spécifiquement l'une à l'autre. De plus, une première séquence nucléotidique peut s'hybrider sur un ou plusieurs segments d'une seconde séquence nucléotidique de telle sorte que les segments intermédiaires ou adjacents ne sont pas impliqués dans l'hybridation. Dans certaines formes de mise en oeuvre de l'invention, une première séquence nucléotidique ou une partie spécifique de celle-ci, est au moins 70%, 80%, 85%, 86%, 87%, 88%, 89%, 90%, 91%, 92%, 93%, 94%, 95%, 96%, 97%, 98%, 99% ou 100% complémentaire à une deuxième séquence nucléotidique ou une partie spécifiée de celle-ci.

Dans l'oligonucléotide à double structure tige-boucle mis en oeuvre dans l'invention, le nucléotide à l'extrémité 5' de la portion F1c est lié, au moyen de liaisons hydrogène, avec le nucléotide correspondant dans la portion F1 et le nucléotide à l'extrémité 3' de la portion B1 est lié, au moyen de liaisons hydrogène, avec le nucléotide correspondant dans la portion B1c.

Typiquement, dans l'oligonucléotide à double structure tige-boucle mis en oeuvre dans l'invention, la portion F1 et la portion F1c comprennent, chacune, de 10 à 35 nucléotides, notamment entre 15 et 25 nucléotides et, à titre d'exemple particulier, 21 nucléotides. La température de fusion (Tm) de F1-F1c est notamment supérieure à la température utilisée lors de l'amplification et, en particulier, supérieure de 5°C à 7°C. L'homme du métier pourra trouver des informations additionnelles quant à ces portions dans [1].

Typiquement, la portion B1 et la portion B1c comprennent, chacune, de 10 à 35 nucléotides, notamment entre 15 et 30 nucléotides et, à titre d'exemple particulier, 25 nucléotides. La température Tm de B1-B1c est notamment supérieure à la température utilisée lors de l'amplification et, en particulier, supérieure de 5°C à 7°C. L'homme du métier pourra trouver des informations additionnelles quant à ces portions dans [1].

En particulier, la portion F2 comprend de 8 à 25 nucléotides et, à titre d'exemple particulier, 18 nucléotides et la portion F0' est telle que la boucle F0 comprend de 10 à 70 nucléotides.

De plus, la portion B2c comprend de 8 à 35 nucléotides, notamment entre 15 et 30 nucléotides et, à titre d'exemple particulier, 18 nucléotides et la portion B0' est telle que la boucle B0 comprend de 10 à 70 nucléotides.

Dans l'oligonucléotide de formule (I), la portion Int peut comprendre jusqu'à 70 nucléotides.

Avantageusement, l'oligonucléotide à double structure tige-boucle mis en oeuvre dans le procédé selon la présente invention comprend moins de 200 nucléotides, notamment moins de 180 nucléotides, en particulier moins de 170 nucléotides, plus particulièrement moins de 160 nucléotides et tout particulièrement moins de 150 nucléotides. Avec une telle taille, la synthèse chimique de cet oligonucléotide est facilitée et le coût de cette synthèse et, par conséquent, le coût du procédé selon l'invention sont moindres.

L'oligonucléotide à double structure tige-boucle mis en oeuvre dans le procédé selon la présente invention est capable de se lier à l'analyte à détecter. En d'autres termes, la séquence nucléotidique de cet oligonucléotide présente au moins une séquence spécifique de l'analyte à détecter, comme, par exemple, un aptamère ou un facteur de transcription ou au moins une séquence complémentaire à une partie de la séquence nucléotidique de l'analyte à détecter lorsque ce dernier est une molécule nucléotidique telle que précédemment définie. Cette séquence i.e. la séquence spécifique de l'analyte à détecter ou la séquence complémentaire à une partie de la séquence nucléotidique de l'analyte peut se trouver dans l'une quelconque des portions de l'oligonucléotide telles que précédemment définies ou dans plusieurs de ces portions. A titre d'exemples, cette séquence peut se trouver dans la portion F1, dans la portion F1c, dans la portion B1, dans la portion B1c, dans la portion F0', dans la portion F2, dans la portion Int, dans la portion B2c, dans la portion B0, à la fois dans la portion F1c et la portion F2, à la fois dans la portion F2 et dans la portion F0', à la fois dans la portion F0' et dans la portion F1, à la fois dans la portion F1c et dans la portion Int (oligonucléotide de formule (I)), à la fois dans la portion F1c et dans la portion B1 (oligonucléotide de formule (II)), à la fois dans la portion Int et dans la portion B1c (oligonucléotide de formule (I)), à la fois dans la portion B1c et dans la portion B0', à la fois dans la portion B0' et dans la portion B2c, à la fois dans la portion B2c et dans la portion B1. Comme précédemment évoqué, l'oligonucléotide à double structure tige-boucle peut comprendre plusieurs séquences spécifiques d'un ou plusieurs analyte(s) à détecter et/ou plusieurs séquences complémentaires à une partie de la séquence nucléotidique d'un ou plusieurs analyte(s) à détecter. Ces différentes séquences peuvent se trouver, dans l'oligonucléotide, dans une même portion ou dans des portions différentes telle(s) que précédemment définie(s).

De plus, dans une forme de mise en oeuvre particulière, l'oligonucléotide à double structure tige-boucle peut comprendre au moins une séquence spécifique de la sonde utilisée pour lier l'analyte à détecter ou au moins une séquence complémentaire à une partie de la séquence nucléotidique de la sonde lorsque cette dernière est une molécule nucléotidique telle que précédemment définie. Dans cette forme de mise en oeuvre notamment utilisable pour des miARN et des petites molécules, l'oligonucléotide se lie au couple analyte-sonde permettant de fait d'avoir une accroche spécifique plus forte. Il est évident que l'oligonucléotide ne doit pas, à la température choisie pour la mise en contact, seulement s'accrocher à la sonde et ce, pour éviter des faux positifs. De même, la ou les séquence(s) spécifique(s) de la sonde ou la ou les séquence(s) complémentaire(s) à une partie de la séquence nucléotidique de la sonde lorsque cette dernière est une molécule nucléotidique doi(ven)t être différente(s) de la ou des séquence(s) spécifique(s) de l'analyte à détecter et de la ou des séquence(s) complémentaire(s) à une partie de la séquence nucléotidique de l'analyte lorsque ce dernier est une molécule nucléotidique. Cette forme de mise en oeuvre particulière est illustrée aux points V.2. et V.3. ci-après. Tout ce qui a été précédemment précisé pour les séquences spécifiques de l'analyte à détecter et les séquences complémentaires à une partie de la séquence nucléotidique de l'analyte et notamment leur localisation dans la séquence de l'oligonucléotide s'applique *mutatis mutandis* aux séquences spécifiques de la sonde et aux séquences complémentaires à une partie de la séquence nucléotidique de la sonde.

Lors de l'étape ii) du procédé selon la présente invention, la surface du support solide sur laquelle est éventuellement maintenu au moins un analyte au moyen d'une sonde est mise en contact avec une solution contenant au moins un oligonucléotide à double structure tige-boucle tel que précédemment défini. Cette solution est avantageusement une solution aqueuse permettant l'interaction haltère/analyte. Elle peut comporter un ou plusieurs des composants suivants : un tampon comme un tampon Tris, phosphate, acétate ou borate ; des sels comme KCI, NaCl, (NH₄)₂SO₄, MgCb ou CaClz ; des détergents ou agents tensioactifs comme Tween^{®}, Triton^{®} ou dodécylsulfate de sodium (ou SDS) ; des agents dénaturants comme formamide ou diméthylsulfoxyde ; un solvant organique comme éthanol, méthanol ou acétonitrile ; et des agents pontants comme formaldéhyde ou glutaraldéhyde. Avantageusement, lorsque l'oligonucléotide comprend une séquence aptamère pour la reconnaissance de l'analyte, la solution utilisée sera composée des éléments en totalité ou en partie constituant le tampon de sélection de l'aptamère. La solution utilisée lors de l'étape ii) peut, en outre, comprendre un ou plusieurs élément(s) choisi(s) parmi un ADN génomique comme de l'ADN simple brin et notamment de l'ADN simple brin de sperme de saumon, du sérum, de l'albumine, un polymère de synthèse, un agent bloquant comme l'agent bloquant Denhardt et tout autre élément permettant de limiter l'adsorption non spécifique. Un exemple particulier de solution mise en oeuvre lors de l'étape ii) est une solution saline contenant 0,02 M de PBS, 1,074 M de NaCl, 0,3% de détergent Tween20, 20 µg/mL d'ADN de sperme de saumon ainsi que 4% d'agent bloquant Denhardt.

Par ailleurs, l'oligonucléotide à double structure tige-boucle est présent dans cette solution en une quantité comprise entre 1 aM et 1 mM, et notamment entre 100 aM et 1 µM. A titre d'exemples particuliers de concentrations utilisables, on peut citer une concentration de 10 pM, de 100 pM, de 1 nM, ou de 10 nM. Avantageusement, l'oligonucléotide à double structure tige-boucle est présent dans la solution mise en oeuvre lors de l'étape ii) en une concentration de 100 pM.

Typiquement, l'étape ii) de mise en contact peut durer entre 1 min et 24 h, notamment entre 2 min et 12 h et, en particulier, entre 5 min et 6 h. Dans une forme de mise en oeuvre particulière, cette mise en contact peut durer entre 10 min et 3 h, entre 15 min et 2 h, entre 20 min et 1 h et, notamment, de l'ordre de 30 min (i.e. 30 min ± 5 min). Dans une autre forme de mise en oeuvre, cette mise en oeuvre peut durer entre 30 min et 6 h, entre 1 h et 5 h, entre 2 h et 4 h et, notamment, de l'ordre de 3 h (i.e. 3 h ± 15 min). De même, l'étape (ii) de mise en contact peut être réalisée à une température comprise entre 4°C et 100°C, notamment entre 10°C et 60°C, en particulier entre 15 et 30°C et, plus particulièrement, à température ambiante (i.e. 23°C ± 5°C) ou à température physiologique (i.e. 37°C ± 5°C).

Suite à l'étape ii) et préalablement à l'amplification LAMP, la surface du support solide doit être lavée de façon à éliminer tous les éléments susceptibles de donner de faux positifs, comme, par exemple, des oligonucléotides à double structure tige-boucle non spécifiquement liés à l'analyte à détecter ou liés à des éléments non liés aux sondes fonctionnalisant la surface du support solide. Il s'agit là de l'étape iii) du procédé selon la présente invention.

Durant cette dernière, la surface du support solide est soumise à au moins un rinçage qui est typiquement effectué avec une solution de rinçage qui est constituée d'une solution aqueuse conservant l'interaction haltère/analyte ou liant irréversiblement l'analyte et l'haltère. Cette solution peut également permettre d'éliminer les interactions non spécifiques. Elle peut comporter un ou plusieurs des composants suivants : un tampon comme un tampon Tris, phosphate, acétate ou borate ; des sels comme KCI, NaCl, (NH₄)₂SO₄, MgCl₂ ou CaCl₂ ; des détergents ou agents tensioactifs comme Tween^{®}, Triton^{®} ou dodécylsulfate de sodium (ou SDS) ; des agents dénaturants comme formamide ou diméthylsulfoxyde ; un solvant organique comme éthanol, méthanol ou acétonitrile ; et des agents pontants comme formaldéhyde ou glutaraldéhyde et un ou plusieurs élément(s) choisi(s) parmi un ADN génomique comme de l'ADN simple brin et notamment de l'ADN simple brin de sperme de saumon, du sérum, de l'albumine et un polymère de synthèse. Ce rinçage peut être répété deux fois, trois fois, cinq fois, 10 fois et voire 50 fois en utilisant, à chaque rinçage, une solution de rinçage identique ou différente. Typiquement, le rinçage est répété trois fois avec une solution comprenant du PBS et 0,1% de Tween^{®}. En variante, le rinçage est répété trois fois avec une solution saline contenant 0,02 M de PBS, 1,074 M de NaCl et 0,3% de détergent Tween20.

De plus, l'étape iii) est réalisée à une température comprise entre 4°C et 100°C, notamment entre 15°C et 80°C, en particulier entre 30°C et 60°C et, plus particulièrement, à température de l'ordre de 40°C (i.e. 40°C ± 5°C) ou à température physiologique.

L'étape iv) du procédé selon l'invention est l'amplification LAMP à deux amorces à proprement parler. Les deux seules amorces mises en oeuvre lors de cette étape répondent respectivement à la formule (III) et à la formule (IV) ci-après :

5'-F1c-F2-3' (III)

5'-B1c-B2-3' (IV)

La portion B2 présente une séquence nucléotidique complémentaire à la séquence nucléotidique de la portion B2c, les portions F1c, F2, B1c et B2c étant telles que précédemment définies.

Les amorces de formule (III) ou de formule (IV) sont respectivement appelées, dans l'amplification LAMP à quatre amorces, amorce FIP (pour « Forward Inner Primer ») et amorce BIP (pour « Backward Inner Primer »).

La température Tm de B2-B2c est notamment supérieure à la température utilisée lors de l'amplification et, en particulier, supérieure de 5°C à 7°C. L'homme du métier pourra trouver des informations additionnelles quant à ces portions dans [1].

La température Tm de F2-F2c est notamment supérieure à la température utilisée lors de l'amplification et, en particulier, supérieure de 5°C à 7°C. L'homme du métier pourra trouver des informations additionnelles quant à ces portions dans [1].

Il convient de noter que, dans l'étape iv) du procédé selon la présente invention, l'analyte n'est pas utilisé comme une amorce de l'amplification LAMP.

L'étape iv) du procédé selon la présente invention consiste à mettre en contact la surface sur laquelle est/sont immobilisée(s) une ou des sonde(s) spécifique(s) de l'analyte à détecter avec un mélange réactionnel comprenant les amorces FIP et BIP et tout élément nécessaire à la production d'un produit d'amplification si l'oligonucléotide à double structure tige-boucle est présent.

Typiquement, le mélange réactionnel mis en oeuvre lors de l'étape iv) comprend un tampon approprié, comme, par exemple, un tampon phosphate ou un tampon Tris ; des désoxyribonucléosides (dNTP), comme, un mélange équimolaire de dATP, dCTP, dGTP et dTTP et une enzyme catalysant l'amplification LAMP. Une telle enzyme est une polymérase présentant une activité de déplacement de brin élevée en plus d'une activité de réplication. A titre d'exemples particuliers de telles enzymes, on peut utiliser l'une des enzymes listées ci-dessous :
- Bst DNA polymerase,
- Bca (exo-) DNA polymerase,
- Klenow fragment of DNA polymerase I,
- Vent DNA polymerase,
- Vent(Exo-) DNA polymerase (exonuclease activity-free Vent DNA polymerase),
- DeepVent DNA polymerase,
- DeepVent(Exo-) DNA polymerase (DeepVent DNA polymerase sans activité exonucléase),
- 29 phage DNA polymerase,
- MS-2 phage DNA polymerase,
- Z-Taq DNA polymerase (Takara Shuzo), et
- KOD DNA polymerase (TOYOBO).

Le milieu réactionnel peut comprendre, en outre, des sels comme des sels de magnésium, des sels de manganèse et/ou sels d'ammonium ; des détergents et/ou des éléments utiles pour la détection et la quantification du produit d'amplification de l'oligonucléotide à double structure tige-boucle comme, par exemple, de la calcéine, un colorant intercalant comme, par exemple, iodure de propidium, SYTO 9, le vert SYBR et l'EvaGreen.

L'étape iv) du procédé selon la présente invention est mise en oeuvre pendant une période de temps et à une température suffisantes pour la production d'un produit d'amplification si l'oligonucléotide à double structure tige-boucle est présent. Avantageusement, cette étape est effectuée dans des conditions isothermes et notamment à une température comprise entre 15°C et 90°C, en particulier, comprise entre 50°C et 80°C et, plus particulièrement de l'ordre de 65°C (i.e. 65°C ± 5°C). La durée de l'étape iv) du procédé selon la présente invention est comprise entre 1 min et 120 min, notamment entre 5 min et 90 min et, en particulier, entre 15 min et 60 min.

L'étape v) consiste à détecter et éventuellement à quantifier le produit d'amplification de l'oligonucléotide à double structure tige-boucle et donc à détecter et éventuellement à quantifier l'analyte auquel l'oligonucléotide à double structure tige-boucle est lié puisque l'accumulation du produit d'amplification est un indicateur de la présence de cet analyte immobilisé à la surface du support solide.

L'étape v) peut être réalisée simultanément à l'étape iv) ou après cette dernière. L'homme du métier connaît différentes techniques pour réaliser cette détection.

Le produit d'amplification peut être détecté, lors de l'étape v) par mesure de la turbidité causée par le pyrophosphate de magnésium précipité en solution comme sous-produit de l'amplification. Cette mesure peut être réalisée à l'oeil nu ou via un turbidimètre.

Le produit d'amplification peut être détecté, lors de l'étape v), par une sonde pH permettant de suivre l'acidification du mélange réactionnel lors de l'amplification LAMP.

Le produit d'amplification peut être détecté, lors de l'étape v), via une électrophorèse sur gel.

Le produit d'amplification peut également être détecté, lors de l'étape v), par un test colorimétrique ou par mesure de fluorescence notamment lorsque la calcéine ou un colorant intercalant fluorescent est présent dans le mélange réactionnel.

Lorsque l'on souhaite réaliser une quantification de l'analyte lors de l'étape v) du procédé selon la présente invention, cette quantification est réalisée par une comparaison avec une gamme étalon d'analyte à des concentrations connues. Cette quantification est une étape classique de la méthode ELISA. Cette quantification peut également mettre en oeuvre des quantités connues d'analyte à détecter, ajoutées à l'échantillon liquide comme précédemment envisagé (échantillon liquide dopé).

Il est décrit aussi, mais ne faisant pas partie de l'invention, un oligonucléotide à double structure tige-boucle susceptible d'être mis en oeuvre dans le procédé selon la présente invention, ledit oligonucléotide répond à la formule (II) ci-après :

5'-F1c-F2-F0'-F1-B1c-B0'-B2c-B1-3' (II)

dans laquelle la portion F1, la portion F2, la portion F0', la portion F1, la portion B1c, la portion B0', la portion B2c et la portion B1 sont telles que précédemment définies.

### BRÈVE DESCRIPTION DES DESSINS

La Figure 1 présente le schéma de principe de la détection d'un ADN conformément au procédé selon l'invention dans lequel le support solide est une bille magnétique.
La Figure 2 présente les courbes d'amplification de la cible ADN selon le protocole décrit au point II.1. Les solutions « réaction 1 » et « réaction 2 » sont des dupliquas contenant les billes, la sonde ADN à 100 nM, la cible à 100 nM et l'haltère à 100 pM. Le contrôle négatif contient les billes et l'haltère à 100 pM avec rinçage. Les contrôles positifs « 100 pM » et « 1 pM » contiennent les billes et l'haltère sans la cible, et aucun rinçage n'a été effectué.

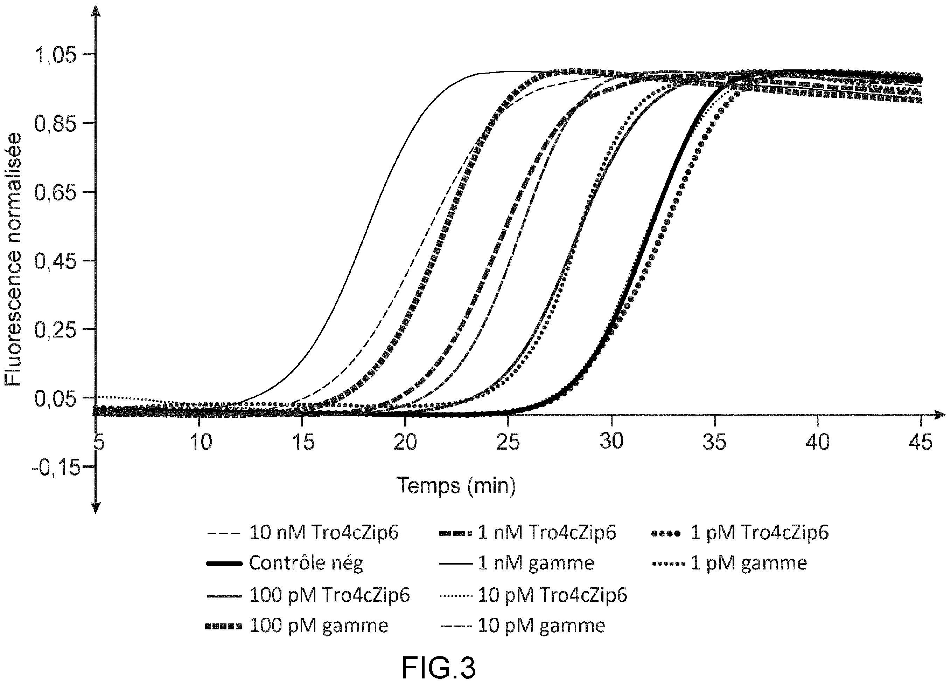

La Figure 3 présente les courbes d'amplification de la cible ADN selon le protocole décrit au point II.1 avec une gamme d'amplification. Les courbes en traits pointillés dont l'intitulé comprend « Tro4cZip6 » représentent les solutions contenant les billes, la sonde ADN, la cible ADN à des concentrations variant de 10 nM à 1 pM et l'haltère à 100 pM. Le contrôle négatif « contrôle nég » contient les billes et l'haltère à 100 pM sans la cible. Les contrôles positifs sont représentés par les courbes dont l'intitulé comprend « gamme ». Ils contiennent les billes, une sonde complémentaire d'une partie de l'haltère et l'haltère.
La Figure 4 présente les courbes d'amplification de la cible ADN selon le protocole décrit au point II.2. Les solutions « réaction a » et « réaction b » sont des dupliquas contenant les billes, la sonde ADN à 100 nM, la cible d'ADN à 100 nM et l'haltère à 100 pM. Le contrôle négatif contient les billes et l'haltère à 100 pM avec rinçage. Les contrôles positifs « 100 pM » et « 100 fM » contiennent les billes et l'haltère sans la cible 3, et aucun rinçage n'a été effectué.

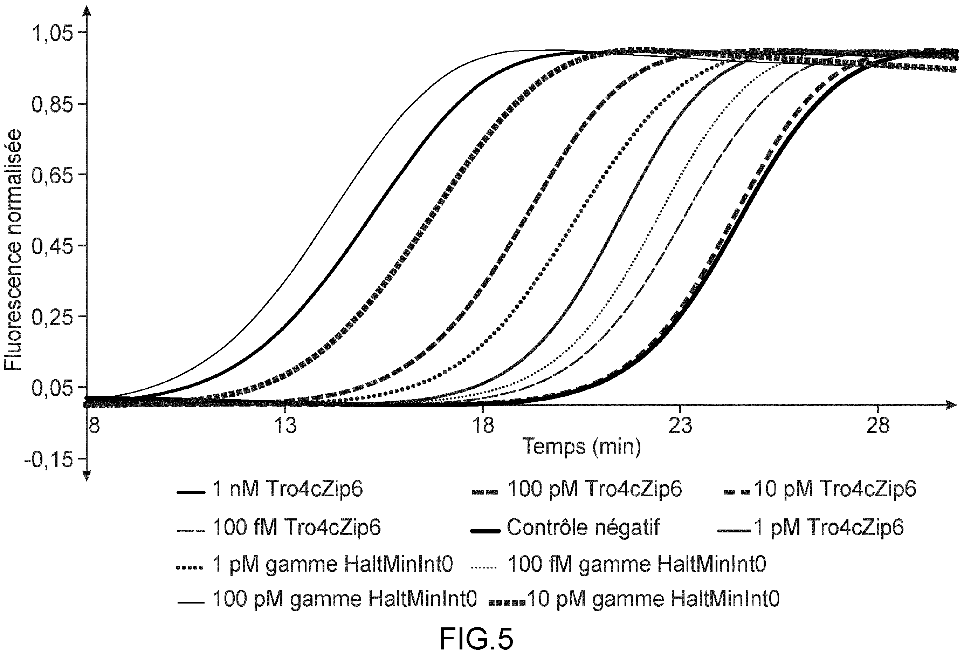

La Figure 5 présente les courbes d'amplification de la cible ADN avec une gamme d'amplification selon le protocole III. Les courbes en traits pointillés dont l'intitulé comprend « Tro4cZip6 » représentent les solutions contenant les billes, la sonde ADN, la cible ADN à des concentrations variant de 1 nM à 100 fM et l'haltère ne contenant aucun nucléotide dans la portion Int à 100 pM. Le contrôle négatif contient les billes et l'haltère à 100 pM sans la cible. Les contrôles positifs sont représentés par les courbes dont l'intitulé comprend « gamme ». Ils contiennent les billes, une sonde complémentaire d'une partie de l'haltère et l'haltère.
La Figure 6 présente la variation du logarithme de la concentration d'ADN cible en fonction du cycle de seuil (Ct). En noir sont représentés les points expérimentaux des contrôles positifs de la Figure 5. En gris sont représentés les points expérimentaux de la gamme de détection contenant l'ADN cible Tro4cZip6.
La Figure 7 présente le schéma de principe de la détection d'une protéine conformément au procédé selon l'invention dans lequel le support solide est une bille magnétique.
La Figure 8 présente les courbes d'amplification de la détection de la troponine. Les solutions « troponine 100 nM » et « troponine 10 nM » contiennent les billes, la sonde anticorps à 100 nM, la troponine à 100 nM et 10 nM respectivement et l'haltère à 100 pM. Le contrôle négatif contient les billes et l'haltère à 100 pM avec rinçage.
La Figure 9 présente le schéma de principe de la détection d'une protéine conformément au procédé selon l'invention dans lequel le support solide est une bille magnétique et deux aptamères spécifiques de la protéine sont utilisées.
La Figure 10A présente la structure de l'aptamère Thr1 utilisé pour détecter la thrombine conformément au procédé selon l'invention dans lequel deux aptamères sont utilisés.
La Figure 10B présente la structure de l'aptamère Thr2 utilisé pour détecter la thrombine conformément au procédé selon l'invention dans lequel deux aptamères sont utilisés.
La Figure 10C présente la structure de l'haltère DTRC du type oligonucléotide à double structure tige-boucle répondant à la formule (I) dans laquelle l'aptamère Thr1 est incorporé dans la portion Int (boucle centrale).
La Figure 11 présente la variation des cycles seuils en fonction de la concentration de protéine cible, obtenus lors de la gamme de détection de la thrombine.
La Figure 12 présente le schéma de principe de la détection d'un mi-ARN conformément au procédé selon l'invention dans lequel le support solide est une bille magnétique.
La Figure 13 présente le schéma de principe de la détection d'une petite molécule conformément au procédé selon l'invention dans lequel le support solide est une bille magnétique.
La Figure 14 présente la variation des cycles seuils en fonction de la concentration d'ADN cible, obtenus lors de la gamme de détection de la cible ADN en plasma.
La Figure 15 présente la variation des cycles seuils en fonction de la concentration d'ADN cible, obtenus lors de la gamme de détection de la cible ADN en sang total dopé puis centrifugé.

### EXPOSÉ DÉTAILLÉ DE MODES DE RÉALISATION PARTICULIERS

### I. Expériences préliminaires.

Les inventeurs ont réalisé une première expérience comparant l'amplification LAMP à 4 amorces d'un brin d'ADN linéaire de 200 bases et l'amplification à 2 ou 4 amorces d'un oligonucléotide à deux structures tige-boucle.

Les résultats ont montré, d'une part, l'absence de quantification possible par une amplification LAMP à quatre amorces d'un brin d'ADN linéaire de 200 bases et, d'autre part, la rapidité de l'amplification LAMP à deux amorces d'un oligonucléotide à deux structures tige-boucle comparée à une amplification LAMP à quatre amorces.

De plus, les inventeurs ont étudié l'amplification LAMP à deux amorces de solutions d'un même oligonucléotide à deux structures tige-boucle à différentes concentrations (100 pM, 10 pM, 1 pM, 100 fM, 10 fM, 1 fM et 100 aM). Par ce travail, il a été montré que l'amplification LAMP à deux amorces fonctionne jusqu'à une concentration d'ADN de 100 aM en moins de 60 min.

### II. Procédé de détection d'un ADN selon la présente invention avec un oligonucléotide de formule (I).

### II.1. Site de reconnaissance dans boucle F0 dudit oligonucléotide.

L'ADN cible à détecter désigné Tro4cZip6 correspond à la séquence TAAGAGGGGCAGCGCATGAGAAAGGTTGGCGTACTGCACGGACCGGTATGCGACCTGGTATGC (SEQ ID NO: 1 dans le listage de séquences en annexe).

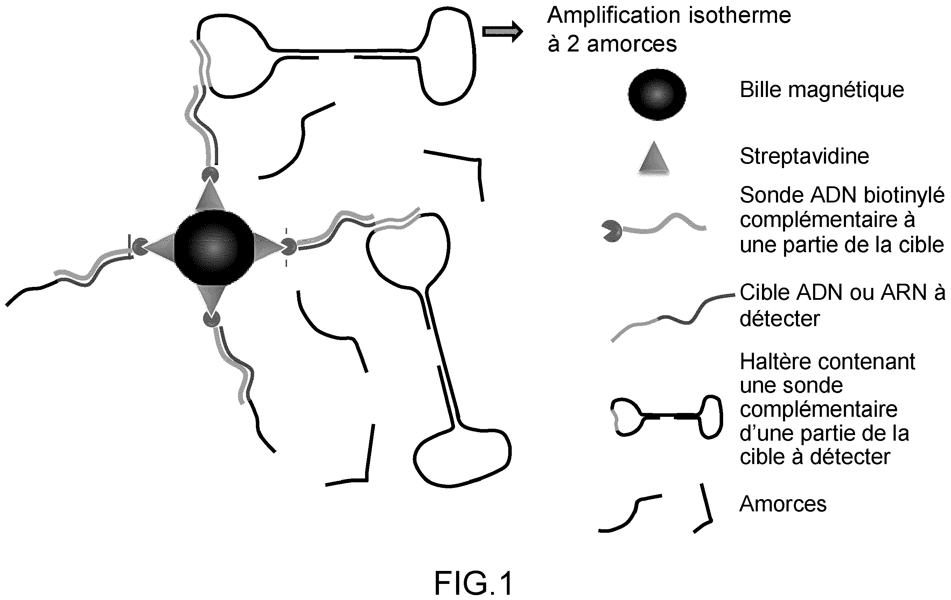

Le schéma de principe de la détection d'ADN selon le procédé de l'invention tel que mis en oeuvre pour détecter cet ADN cible est présenté à la Figure 1.

Des séquences d'ADN sondes biotinylées, désignées Zip6cbio, complémentaires d'une partie de la séquence de l'ADN cible et de séquence CGCATACCAGGTCGCATACCGGTC (SEQ ID NO: 2 dans le listage de séquences en annexe) à 100 nM sont incubées avec 200 picogrammes de microbilles magnétiques recouvertes de streptavidine à 25°C pendant 30 min pour les immobiliser sur les microbilles. Puis, les microbilles sont agrégées au moyen d'un aimant et rincées trois fois avec une solution comprenant du PBS et 0,1% de Tween^{®}.

Ensuite, l'ADN cible à 100 nM est incubé dans la solution contenant les microbilles à 37°C pendant 30 min, et trois rinçages sont effectués comme précédemment.

La troisième étape consiste à incuber, à 37°C, pendant 30 min, 100 pM d'un oligonucléotide à deux structures tige-boucle contenant, dans sa boucle F0, une sonde complémentaire d'une partie de l'ADN cible à détecter. Cet oligonucléotide est de séquence
CGGATCCAAGACGCGGTTTATCGTGCAGTACGCCAACCTTTCTCATGCGCTGCCCCTCTTAATAAA CCGCGTCTTGGATCCGTGACATCTCTGAGTTATATCTTTCCCCCCCTCACTGACCCTCCTTCGGGGG AAAGATATAACTCAGAGATG (SEQ ID NO: 3 dans le listage de séquences en annexe). Puis, les microbilles sont agrégées au moyen d'un aimant et rincées trois fois avec une solution comprenant du PBS et 0,1% de Tween^{®}, à 40°C.

Finalement, après la dernière étape de rinçage, les deux amorces FIP et BIP et l'enzyme Bst 2.0 DNA Polymerase (New England Biolabs) nécessaires à l'amplification LAMP sont ajoutées. L'amorce FIP est de séquence CGGATCCAAGACGCGGTTTATCGTGCAGTACGCCAACCTTTCTCA (SEQ ID NO: 4 dans le listage de séquences en annexe) et l'amorce BIP est de séquence CATCTCTGAGTTATATCTTTCCCCCGAAGGAGGGTCAGTGAGG (SEQ ID NO: 5 dans le listage de séquences en annexe). L'amplification est réalisée à une température de 65°C.

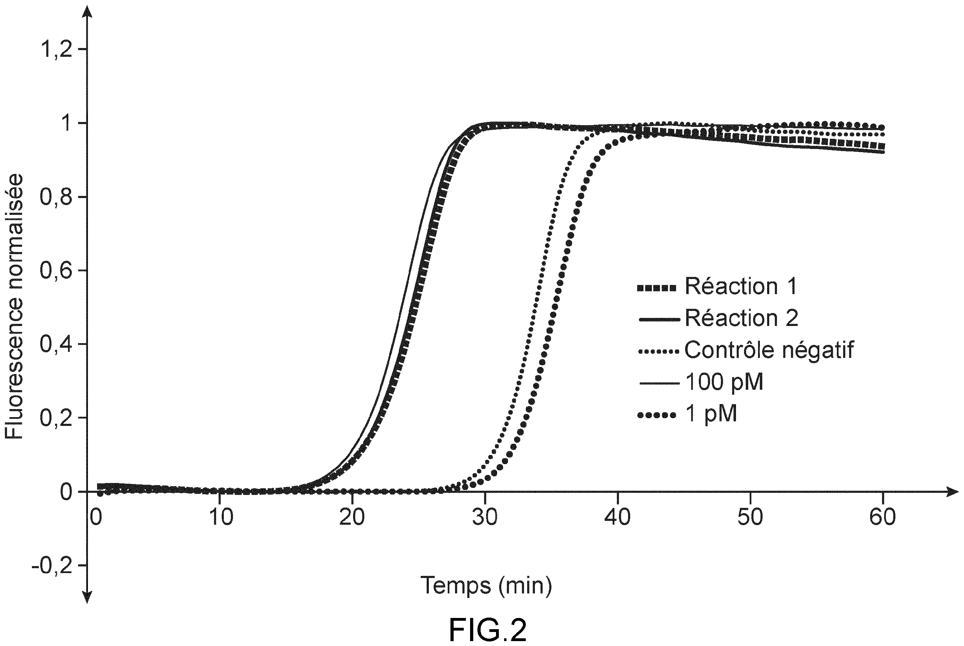

Les résultats de cette amplification sont présentés à la Figure 2.

Les courbes intitulées « réaction 1 » et « réaction 2 » montrent que la détection de la cible ADN est possible en une vingtaine de minutes. En effet, ces courbes sont superposées à la courbe de contrôle positif contenant 100 pM d'haltère, tandis que le contrôle négatif est amplifié à partir de 30 minutes seulement. L'amplification du contrôle négatif est due à des résidus d'haltères qui n'ont pas été entièrement éliminés lors des rinçages. L'écart significatif (plus de 10 minutes) entre les Ct des courbes « réaction » et du contrôle négatif permet d'affirmer que la méthode utilisée permet de détecter une cible ADN dans un milieu tampon.

Cette expérimentation permet donc de valider la détection d'ADN par amplification à deux amorces conformément au procédé selon l'invention.

Des expériences complémentaires ont été réalisées par les inventeurs. Ainsi, une gamme de quantifications a été réalisée pour définir la limite de sensibilité de la méthode. Les courbes d'amplification obtenues sont présentées à la Figure 3.

Les courbes expérimentales présentées en Figure 3 mettent en évidence l'aspect quantitatif de la méthode, d'une part, et la limite de détection atteinte jusqu'à présent, d'autre part. En effet, les courbes obtenues de la gamme réalisée entre 10 nM et 1 pM de cible montrent qu'il est possible de quantifier une cible ADN dans un échantillon à partir d'une gamme étalon simultanément établie, selon le même principe que la gamme étalon d'un test ELISA.

La limite de détection atteinte lors de cette expérience est de 100 pM d'ADN cible. Cependant, différentes optimisations ont été réalisées depuis permettant d'atteindre une limite de détection de 10 pM dans un milieu complexe.

De même, des expériences mettant en oeuvre d'autres sondes et d'autres cibles d'ADN à détecter ont été effectuées afin de valider le caractère générique du procédé.

Enfin, des expériences identiques aux précédentes mais réalisées dans un milieu de type plasma sanguin ont permis de valider la méthode de détection dans un milieu complexe.

### II.2. Site de reconnaissance dans boucle B0 dudit oligonucléotide.

Cette expérience est similaire à l'expérience du point II.1 ci-dessus même sonde d'ADN, même oligonucléotide à double structure tige-boucle et même mode opératoire mais une nouvelle cible d'ADN reconnue par une autre partie de l'oligonucléotide est détectée. Cette cible est de séquence GAAGGAGGGTCAGTGAGGGACCGGTATGCGACCTGGTATGCG (SEQ ID NO: 6 dans le listage de séquences en annexe). Son site de reconnaissance dans l'oligonucléotide se trouve dans la boucle B0.

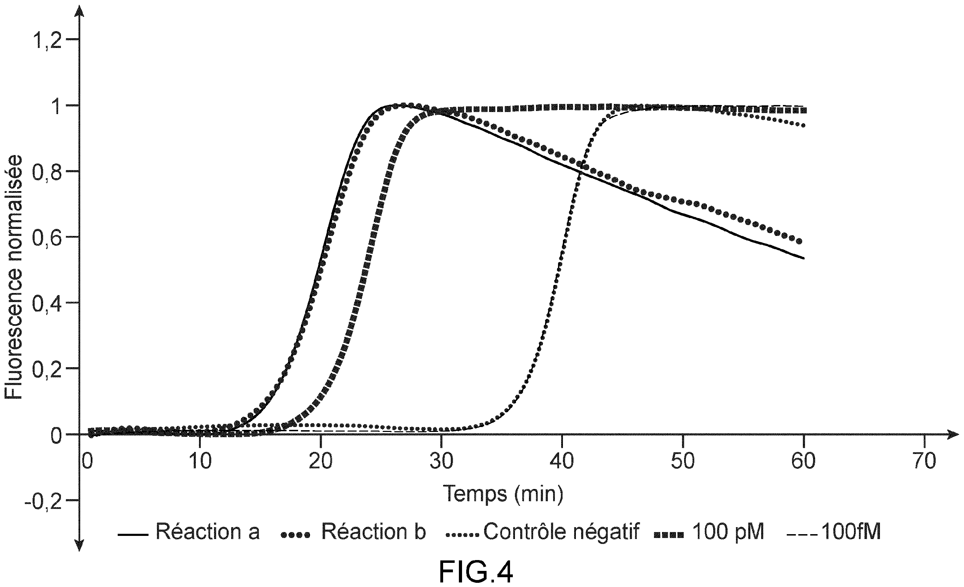

Les résultats obtenus et présentés à la Figure 4 montrent que la position de la sonde complémentaire de la cible dans l'oligomère n'inhibe pas l'amplification à deux amorces.

### III. Procédé de détection d'un ADN selon la présente invention avec un oligonucléotide de formule (II).

Un haltère de formule (II), c'est-à-dire ne contenant pas d'oligonucléotide dans la portion Int, et donc plus court que celui utilisé précédemment de formule (I) a été synthétisé chimiquement.

Cet oligonucléotide désigné HaltMinInt0 est de séquence

CGGATCCAAGACGCGGTTTATCGTGCAGTACGCCAACCTTTCTCAATAAACCGCGTCTTGGATCCG
CATCTCTGAGTTATATCTTTCCCCCCCTCACTGACCCTCCTTCGGGGGAAAGATATAACTCAGAGA
TG (SEQ ID NO: 7 dans le listage de séquences en annexe).

Le procédé de détection réalisé est identique à celui décrit au point II.1. L'ADN sonde est la séquence nommée SEQ ID NO: 2 dans le listage de séquences en annexe. Les amorces FIP et BIP sont identiques à celles décrites au point II.1. La cible à détecter est de séquence nommée SEQ ID NO: 6 dans le listage de séquences en annexe. L'haltère utilisé est celui décrit ci-dessus. Le mode opératoire est identique à celui décrit au point II.1.

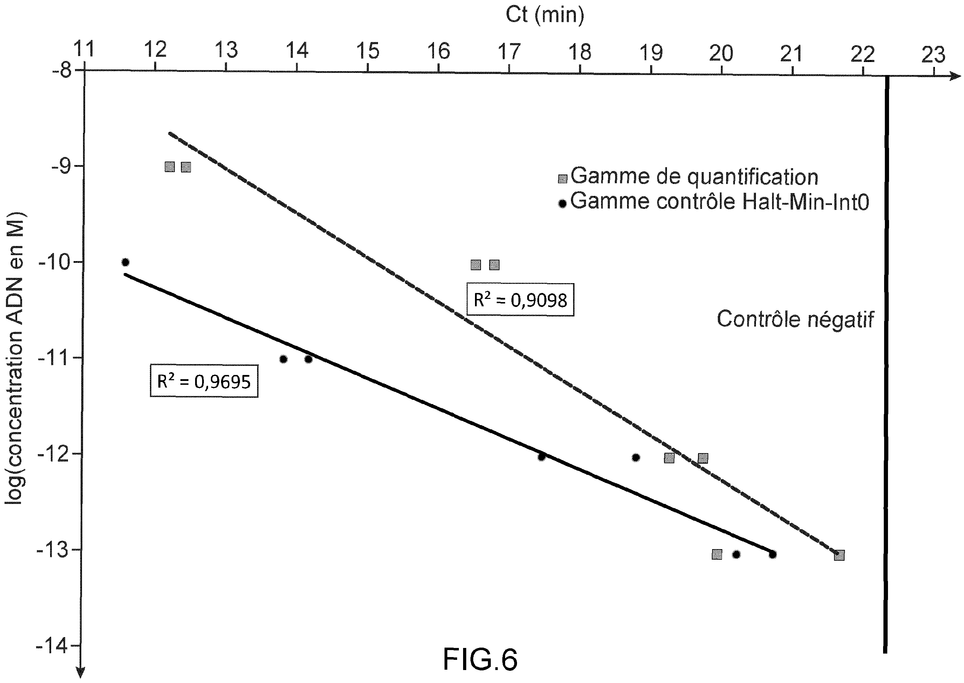

Les courbes d'amplification obtenues sont présentées en Figure 5. Les résultats des Figures 5 et 6 prouvent tout d'abord que l'amplification d'un haltère de formule (II) est fonctionnelle. Ensuite, ils montrent que l'amplification est accélérée du fait de la longueur réduite de l'haltère. La limite de détection atteinte est de 100 pM.

Cette expérimentation permet donc de valider l'affirmation selon laquelle la portion Int est optionnelle dans l'haltère. Des résultats expérimentaux complémentaires ont également permis de confirmer le double intérêt de raccourcir la séquence de l'haltère : optimiser l'étape de fabrication et accélérer la détection.

### IV. Procédé de détection d'une protéine selon la présente invention avec un oligonucléotide de formule (I).

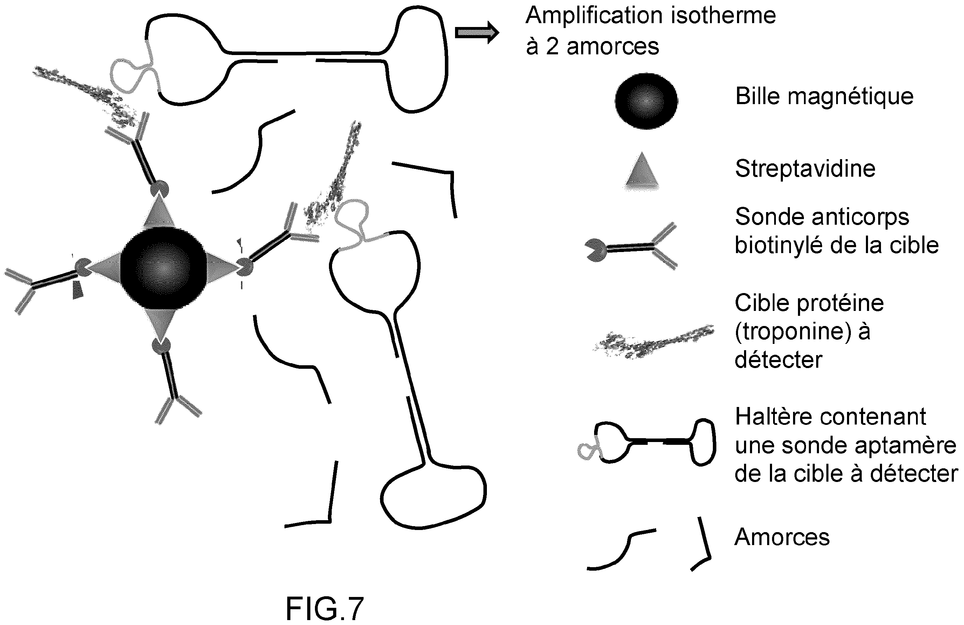

Dans cette expérience, le procédé selon la présente invention est utilisé pour détecter une protéine, la troponine dans un format de type sandwich par amplification LAMP à deux amorces comme schématisé à la Figure 7.

Le même protocole que celui décrit au point II.1 est utilisé mais la première sonde ADN est remplacée par un anticorps anti-troponine biotinylé Anti-h cTnl 9701 SPRN-5 (Medix Biochemica). La cible à détecter est maintenant la troponine et non plus une séquence d'ADN. L'haltère de séquence SEQ ID NO: 3 dans le listage de séquences en annexe contient une sonde aptamère spécifique de la troponine.

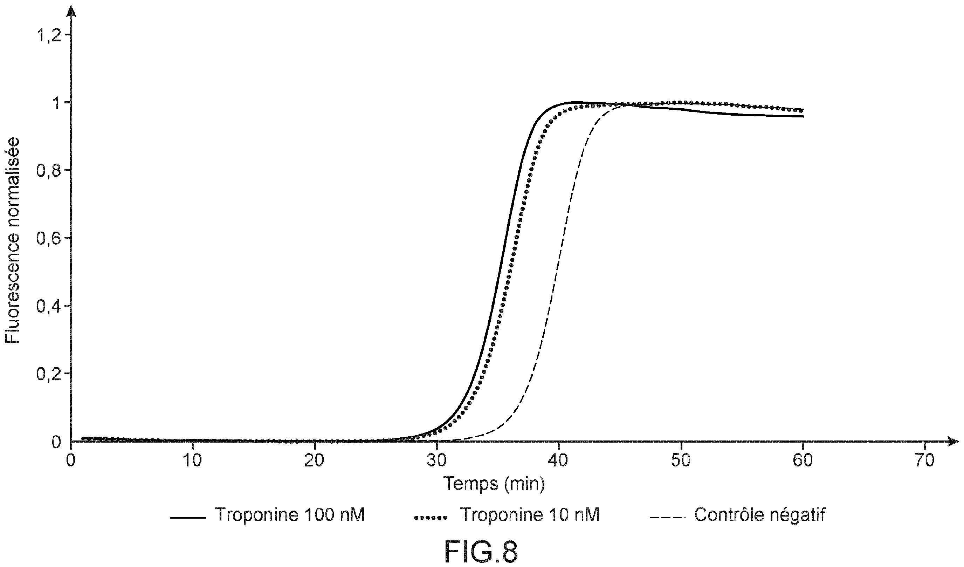

Les résultats obtenus et présentés à la Figure 8 témoignent de la possibilité de la détection de la troponine par le procédé selon la présente invention.

### V. Autres formes de mise en oeuvre du procédé selon la présente invention.

### V.1. Détection de protéines avec deux aptamères.

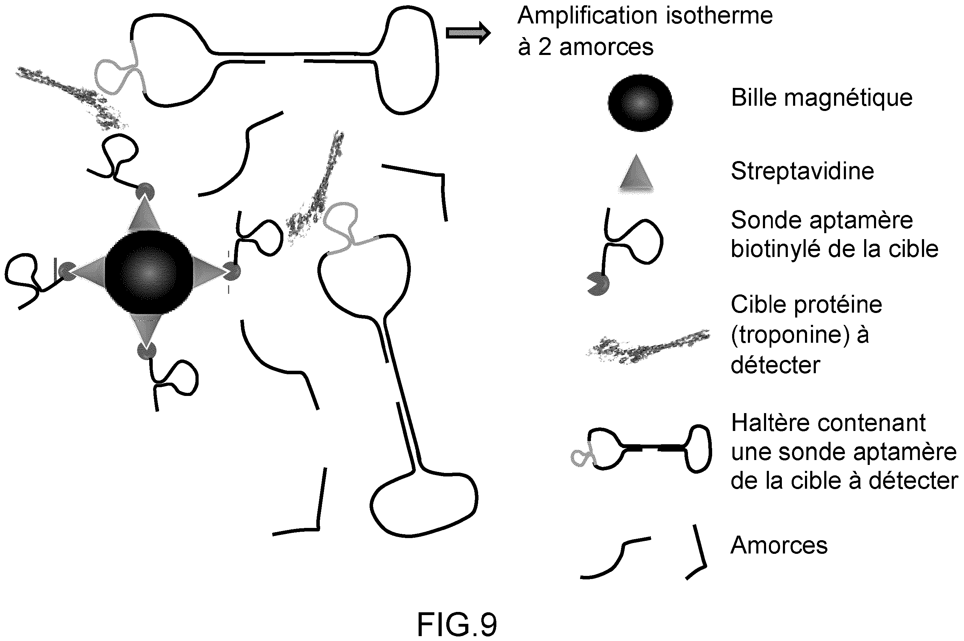

Le procédé de détection selon l'invention peut également être utilisé pour détecter une protéine dans un format de type sandwich avec deux aptamères spécifiques de la cible. Aussi, la sonde anticorps de l'expérience présentée au point IV ci-dessus est remplacée pour un aptamère biotinylé selon le schéma de principe présenté à la Figure 9.

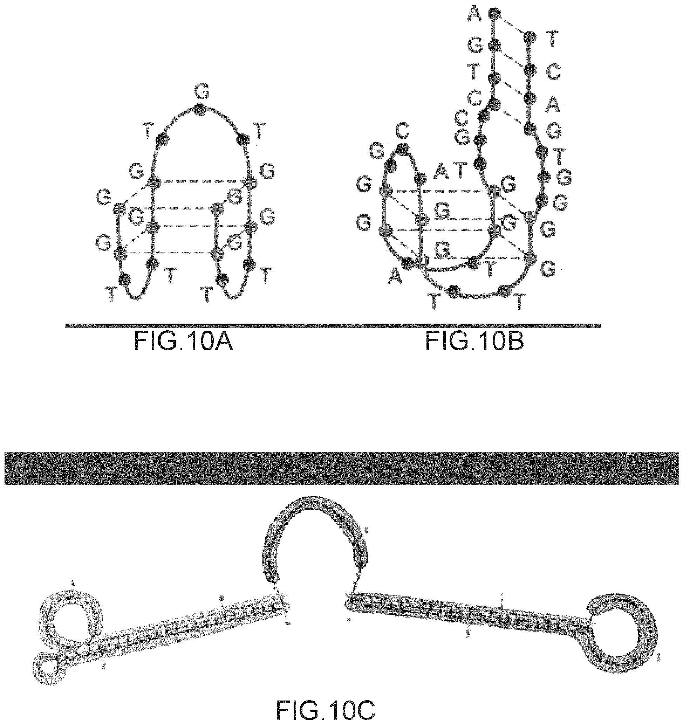

Pour illustrer cette forme de mise en oeuvre, la thrombine est utilisée. En effet, cette dernière peut être détectée en sandwich par deux aptamères que sont Thr1 de séquence 5'-GGTTGGTGTGGTTGG-3' (SEQ ID NO: 8 dans le listage de séquences en annexe) et Thr2 de séquence 5'-AGTCCGTGGTAGGGCAGGTTGGGGTGACT-3' (SEQ ID NO: 9 dans le listage de séquences en annexe). Ces deux aptamères possèdent une conformation en G-quadruplex, correspondant au repliement de 8 nucléotides contenant la base guanine comme schématisé sur les Figures 10A et 10B, cette conformation étant responsable de la reconnaissance de la thrombine.

Une solution à 2 µM de Thr2 biotinylé est incubée avec 200 µg de microbilles magnétiques recouvertes de streptavidine à 25°C pendant 30 min pour les immobiliser sur les microbilles. Après trois lavages, les microbilles sont mises en contact avec du tampon de blocage contenant 5% de BSA et 20 µg/mL d'ADN simple brin de sperme de saumon, pendant 30 min, à 25°C.

Quatre solutions de thrombine à des concentrations comprises entre 100 nM et 100 pM sont incubées avec les microbilles fonctionnalisées moyennant quoi des microbilles revêtues de thrombine sont obtenues.

L'haltère utilisé est l'haltère DTRC tel qu'illustré à la Figure 10C. Ce dernier contient la séquence de l'aptamère Thr1 et est de séquence 5'-CGGATCCAAGACGCGGTTTATCGTGCAGTACGCCAACCTTTCTCAATAAACCGCGTCTTGGATCCG TGGTTGGTGTGGTTGGGACATCTCTGAGTTATATCTTTCCCCCCCTCACTGACCCTCCTTCGGGGG AAAGATATAACTCAGAGATG-3' (SEQ ID NO: 10 dans le listage de séquences en annexe). Cet haltère, préalablement à l'étape ii) du procédé selon l'invention i.e. préalablement à l'incubation avec les microbilles revêtues de thrombine, est dénaturé à 90°C puis lentement refroidi, en vue de favoriser le repliement de l'aptamère Thr1.

L'incubation de microbilles revêtues de thrombine avec 100 pM d'haltère DTRC dans un tampon d'incubation (0,02 M de PBS, 1,074 M de NaCl, 0,3% de détergent Tween20, 20 µg/mL d'ADN de sperme de saumon ainsi que 4% d'agent bloquant Denhardt) est effectuée à 25°C, pendant 30 min. Après trois lavages avec un tampon de lavage contenant 0,02 M de PBS, 1,074 M NaCl et 0,3 % de détergent Tween20), une amplification LAMP à deux amorces est mise en oeuvre selon un mode opératoire identique à celui décrit au point II.1.

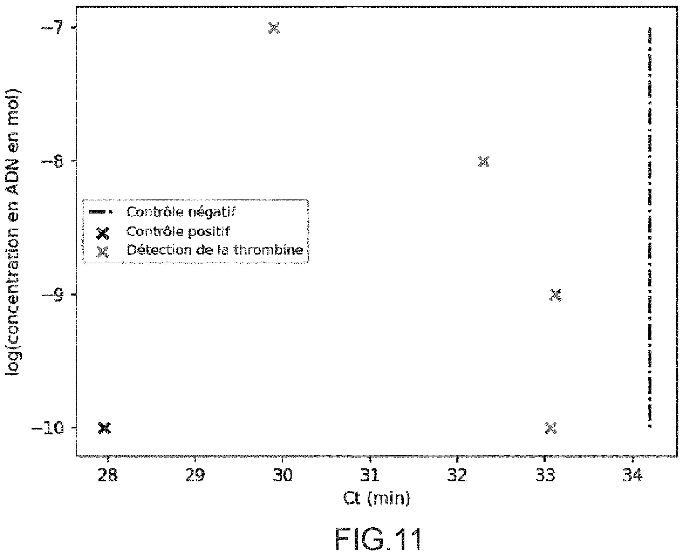

Les résultats sont visibles sur la Figure 11. On observe que la thrombine a bien été détectée. En effet, le contrôle négatif qui comprend l'ensemble des réactifs mais pas la thrombine est détecté en dernier. La gamme de quantification obtenue s'étend de 100 nM à 1 nM. La thrombine à une concentration de 100 pM a été détectée mais le cycle obtenu est égal à celui obtenu pour 1 nM. La détection est donc possible mais la quantification difficile à ces concentrations. Le contrôle positif, qui contient l'haltère DTRC à une concentration de 100 pM, est détecté 5 min avant le point de détection correspondant (contenant 100 pM de thrombine et 100 pM d'haltère DTRC). Cela suggère que la totalité des haltères DTRC incubés avec la thrombine ne s'est pas fixée et/ou que les molécules de thrombine n'ont pas été capturées dans leur totalité.

### V.2. Détection de miARNs.

Une autre cible peut être envisagée : les oligonucléotides ARNs courts ou micro-ARNs (miARNs) comme par exemple les miARNs cardiaques. Comme exposé par Wang *et al*, 2010 [5], ces séquences ARN semblent être des biomarqueurs intéressants pour le diagnostic des infarctus du myocarde.

Le principe est similaire à la détection d'un ADN mais légèrement plus complexe dans la mesure où il s'agit de séquences oligonucléotidiques courtes (entre 20 et 22 nucléotides). Il faut donc établir un sandwich ADN/ARN où le nombre de bases hybridées entre la cible et les sondes est suffisamment important pour garantir la stabilité du complexe. La sonde ADN est donc conçue pour être complémentaire d'une partie de la séquence cible ARN (entre 12 et 16 bases) pour que le complexe formé soit suffisamment stable à température ambiante et puisse subir les rinçages. De même, la sonde haltère contient une séquence de 6 à 10 bases complémentaires de la cible ARN (celles non comprises dans la sonde ADN). La sonde ADN est également complémentaire d'une partie de la sonde haltère (entre 4 et 8 nucléotides complémentaires) ce qui augmente la formation du complexe sonde ADN-miRNA-haltère.

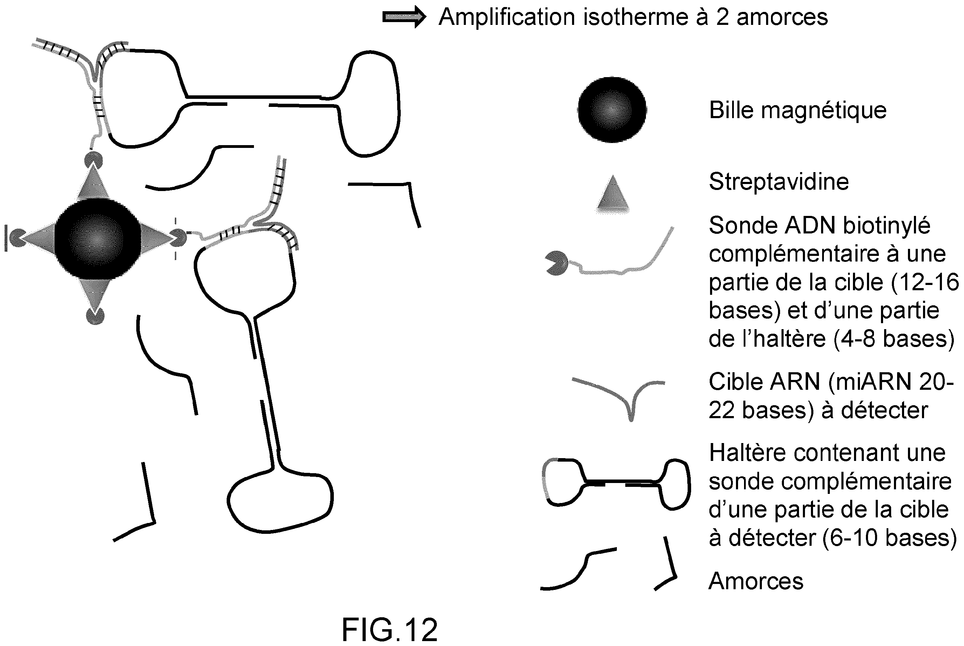

Le schéma de principe de la détection des miARNs selon le procédé de la présente invention est présenté à la Figure 12.

### V.3. Détection de petites molécules.

Un autre type de cible qui peut être visé par le procédé de détection selon l'invention est la catégorie des petites molécules.

En effet, les petites molécules sont difficilement détectables en raison de leur petite taille et de leur présence en faibles concentrations. Les anticorps ne peuvent pas être utilisés comme sonde. Le modèle de détection de l'adénosine décrit par Melaine *et al,* 2016 [6], peut être utilisé.

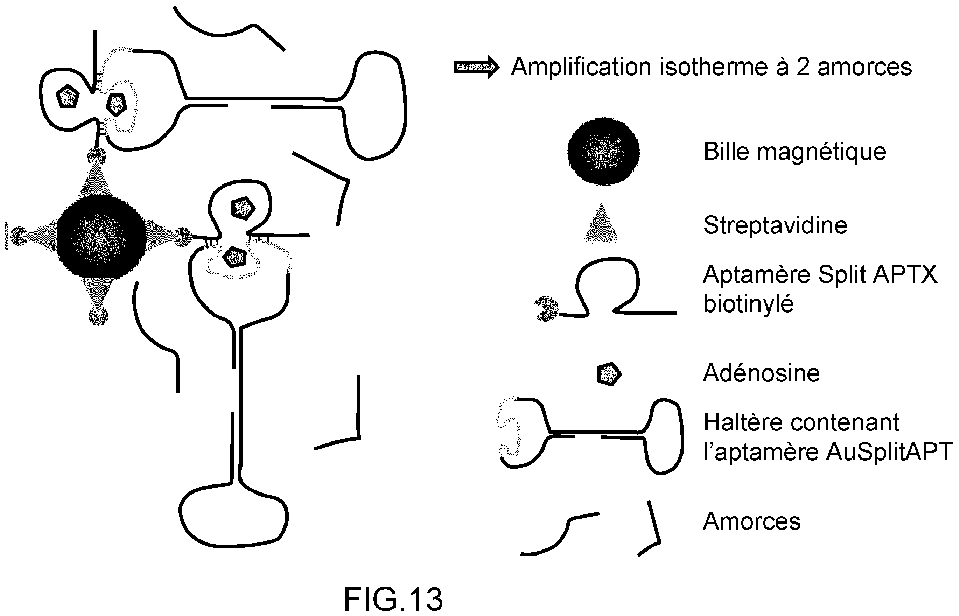

Le schéma de principe de détection de petites molécules selon le procédé de la présente invention est présenté à la Figure 13.

### VI. Détection et quantification en milieu complexe.

### VI.1. Plasma comme milieu complexe.

Cette première expérience consiste à valider le protocole utilisé précédemment en incubant la cible ADN non pas dans une solution tamponnée optimale pour l'hybridation mais dans du plasma sanguin afin d'observer l'effet de matrice.

Ce dernierest récupéré à partir de sang humain fourni par l'Établissement Français du Sang (EFS). Plusieurs tubes sont centrifugés et les plasmas surnageant sont prélevés puis mélangés. Ainsi, le mélange de plasmas issus de plusieurs patients permet d'obtenir une solution homogène et « standardisée » qui n'est pas dépendante du patient. Les expériences sont effectuées en laboratoire de classe 2.

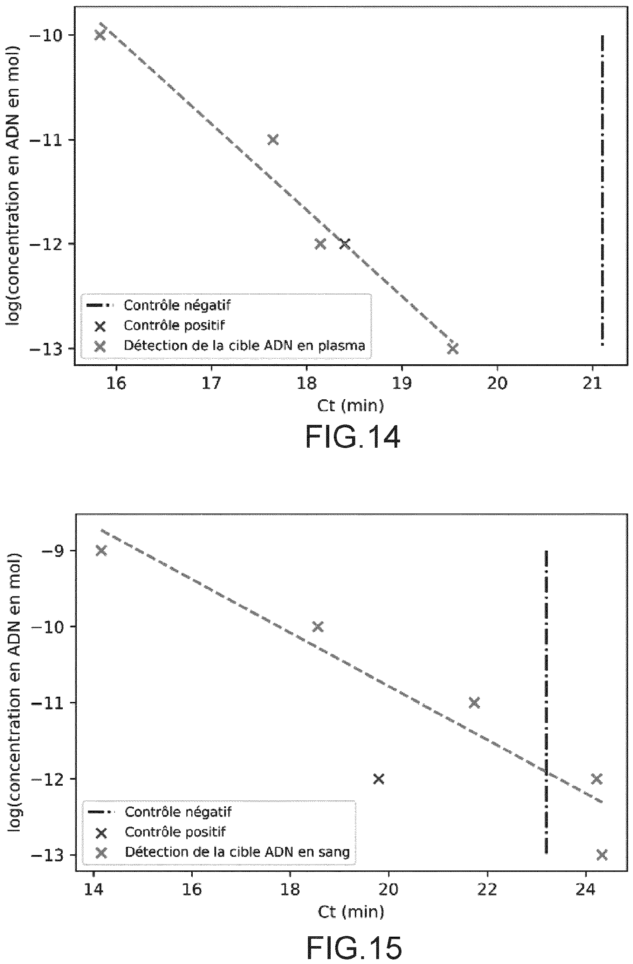

Cinq solutions de plasma dopées avec l'ADN cible Tro4cZip6 (SEQ ID NO: 1 dans le listage de séquences en annexe) à des concentrations comprises entre 100 pM et 100 fM sont incubées avec les billes recouvertes de sondes. Trois rinçages sont effectués puis l'haltère HaltMinInt0 (SEQ ID NO: 7 dans le listage de séquences en annexe) est incubé à 37°C à une concentration de 100 pM. Les résultats des réactions d'amplification sont présentés sur la Figure 14.

Ces résultats sont très satisfaisants dans la mesure où la détection de l'ADN cible dans le plasma fonctionne très bien et sa quantification est possible jusqu'à une concentration de 100 fM : 10⁵ copies d'ADN dans du plasma peuvent donc être détectés en moins de 20 min.

Contrairement à ce qui aurait pu être initialement envisagé, à savoir l'inhibition de la réaction d'amplification par des protéines bloquantes présentes dans le plasma, cette expérience nous permet de conclure que le plasma susceptible d'être utilisé, dans le cadre de la présente invention, en tant qu'échantillon liquide ne perturbe pas la réaction de détection, ni d'amplification. Au contraire, la sensibilité de la détection est meilleure.

### VI.2. Sang total comme milieu complexe.

L'expérience suivante a pour objectif de se rapprocher encore davantage des conditions réelles. En effet, la cible à détecter telle qu'un biomarqueur cardiaque se situe dans le sang du patient. Il s'agit donc ici de doper non pas le plasma mais le sang total avec la même cible ADN, puis de réaliser la détection.

Plusieurs tubes de sang humain du même groupe fournis par l'EFS ont donc été mélangés puis aliquotés. Cinq échantillons sont ensuite dopés avec différentes concentrations de cible ADN comprises entre 1 nM et 100 fM puis centrifugés afin d'en extraire le plasma censé contenir l'ADN à détecter. Le surnageant est récupéré puis incubé avec les billes contenant les sondes. La suite du protocole est identique à celle du point VI.1 ci-dessus.

Les résultats des réactions d'amplification sont présentés sur la Figure 15. La réaction de détection de l'ADN cible a fonctionné jusqu'à 10 pM soit 10⁷ copies. En effet, la droite sur le graphique, représentant le contrôle négatif, apparaît plus tôt que le cycle seuil de la réaction d'amplification de l'ADN cible à 1 pM. Le décalage entre le contrôle positif et le point de détection correspondant à la même concentration est certainement dû à des pertes au cours de l'expérience de détection.

En effet, dans le cas du contrôle positif, 1 pM d'haltère a été incubé avec les billes et s'est ainsi hybridé sur les sondes immobilisées. Dans le cas de la détection, les haltères ont été incubés en excès à une concentration de 100 pM avec la cible théoriquement présente à une concentration de 1 pM. Mais la totalité des cibles ne s'est certainement pas accrochée aux billes. Ainsi, bien que l'haltère soit ajouté en excès, le nombre maximum d'haltères présents et donc amplifiés est égal au nombre de cibles restantes dans la solution, soit moins de 1 pM.

La quantification d'une cible oligonucléotidique en sang total par la méthode LAMP à 2 amorces est donc possible jusqu'à une limite de quantification de 10 pM. A noter toutefois que ces expériences ont été réalisées sans optimisation du procédé afin d'améliorer la sensibilité de la détection.

### Références bibliographiques

[1] Brevet US 6,410,278 publié le 25 juin 2002.
[2] Pourhassan-Moghaddam et al, 2013, « Protein détection through different platforms of immuno-loop-mediated isothermal amplification », Nanoscale Research Letters, vol. 8:485, pages 1-11.
[3] Du et al, 2016, « A ligation-based loop-mediated isothermal amplification (ligation-LAMP) strategy for highly selective microRNA détection », Chem. Commun., vol. 52, pages 12721-12724.
[4] Demande de brevet CN 106148549 publiée le 23 novembre 2016.
[5] Wang et al, 2010, « Circulating microRNA: a novel potential biomarker for early diagnosis of acute myocardial infarction in humans », Clinical Research, vol. 31, pages 659-666.
[6] Melaine et al, 2016, « A nanoparticle-based thermo-dynamic aptasensor for small molécule détection », Nanoscale, vol. 8, page 16947-16954.

## Revendications

1. Procédé pour détecter et éventuellement quantifier un analyte éventuellement présent dans un échantillon liquide, comprenant les étapes suivantes :
i) mettre en contact ledit échantillon liquide avec la surface d'un support solide comprenant au moins une zone active sur laquelle au moins une sonde apte à lier ledit analyte est immobilisée ;
ii) mettre en contact ladite surface avec une solution contenant au moins un oligonucléotide à double structure tige-boucle, capable de se lier audit analyte, ledit oligonucléotide ayant été synthétisé préalablement à ladite mise en contact ;
iii) laver ladite surface ;
iv) mettre en contact ladite surface avec deux amorces d'amplification isotherme médiée par des boucles dans des conditions permettant l'amplification dudit oligonucléotide ;
v) détecter et éventuellement quantifier le produit de l'amplification dudit oligonucléotide.

2. Procédé selon la revendication 1, **caractérisé en ce que** ledit oligonucléotide à double structure tige-boucle répond à la formule (I) ou (II) ci-après :
5'-F1c-F2-F0'-F1-Int-B1c-B0'-B2c-B1-3' (I)
5'-F1c-F2-F0'-F1-B1c-B0'-B2c-B1-3' (II)
dans lesquelles
la portion F1c présente une séquence nucléotidique complémentaire à la séquence nucléotidique de la portion F1 moyennant quoi la portion F1c et la portion F1 s'hybrident pour former la tige de la première structure tige-boucle,
la portion F2-F0' forme la boucle de la première structure tige-boucle,
la portion B1 présente une séquence nucléotidique complémentaire à la séquence nucléotidique de la portion B1c moyennant quoi la portion B1 et la portion B1c s'hybrident pour former la tige de la seconde structure tige-boucle,
la portion B0'-B2c forme la boucle de la seconde structure tige-boucle, et
la portion Int sépare la portion F1 et la portion B1c et comprend au moins un nucléotide.

3. Procédé selon la revendication 2, **caractérisé en ce que**, dans ledit oligonucléotide à double structure tige-boucle de formule (I) ou (II),
ladite portion F1 et ladite portion F1c comprennent, chacune, de 10 à 35 nucléotides ;
ladite portion B1 et ladite portion B1c comprennent, chacune, de 10 à 35 nucléotides ;
ladite portion F2 comprend de 8 à 25 nucléotides et ladite portion F0' est telle que la boucle de la première structure tige-boucle comprend de 10 à 70 nucléotides ; et/ou
ladite portion B2c comprend de 8 à 35 nucléotides et ladite portion B0' est telle que la boucle de la seconde structure tige-boucle comprend de 10 à 70 nucléotides.

4. Procédé selon la revendication 2 ou 3, **caractérisé en ce que**, dans ledit oligonucléotide à double structure tige-boucle de formule (I) ou (II),
ladite portion F1 et ladite portion F1c comprennent, chacune, 21 nucléotides ;
ladite portion B1 et ladite portion B1c comprennent, chacune, 25 nucléotides;
ladite portion F2 comprend 18 nucléotides ; et/ou ladite portion B2c comprend 18 nucléotides.

5. Procédé selon l'une quelconque des revendications 2 à 4, caractérisé en que ledit oligonucléotide à double structure tige-boucle présente au moins une séquence spécifique de l'analyte à détecter ou au moins une séquence complémentaire à une partie de la séquence nucléotidique de l'analyte à détecter lorsque ce dernier est une molécule nucléotidique.

6. Procédé selon la revendication 5, **caractérisé en ce que** ladite séquence spécifique de l'analyte à détecter ou ladite séquence complémentaire à une partie de la séquence nucléotidique de l'analyte se trouve dans la portion F1, dans la portion F1c, dans la portion B1, dans la portion B1c, dans la portion F0', dans la portion F2, dans la portion Int, dans la portion B2c, dans la portion B0', à la fois dans la portion F1c et la portion F2, à la fois dans la portion F2 et dans la portion F0', à la fois dans la portion F0' et dans la portion F1, à la fois dans la portion F1c et dans la portion Int pour un oligonucléotide de formule (I), à la fois dans la portion F1c et dans la portion B1 pour un oligonucléotide de formule (II), à la fois dans la portion Int et dans la portion B1c pour un oligonucléotide de formule (I), à la fois dans la portion B1c et dans la portion B0', à la fois dans la portion B0' et dans la portion B2c, à la fois dans la portion B2c et dans la portion B1.

7. Procédé selon l'une quelconque des revendications 2 à 6, caractérisé en que ledit oligonucléotide à double structure tige-boucle présente au moins une séquence spécifique de ladite sonde ou une séquence complémentaire à une partie de la séquence nucléotidique de la sonde lorsque cette dernière est une molécule nucléotidique.

8. Procédé selon l'une quelconque des revendications 1 à 7, **caractérisé en ce que** les deux amorces mises en oeuvre lors de ladite étape iv) répondent respectivement à la formule (III) et à la formule (IV) ci-après :
5'-F1c-F2-3' (III)
5'-B1c-B2-3' (IV)
La portion B2 présente une séquence nucléotidique complémentaire à la séquence nucléotidique de la portion B2c, les portions F1c, F2, B1c et B2c étant telles que définies dans l'une quelconque des revendications 2 à 4.

9. Procédé selon l'une quelconque des revendications 1 à 8, **caractérisé en ce que** ledit produit d'amplification est détecté, lors de ladite étape v), par mesure de la turbidité, par une sonde pH, via une électrophorèse sur gel, par un test colorimétrique ou par mesure de fluorescence.

10. Procédé selon l'une quelconque des revendications 1 à 9, **caractérisé en ce que**, suite à ladite étape i) et préalablement à ladite étape ii), la surface du support solide est lavée.

## Patentansprüche

1. Verfahren zum Nachweisen und gegebenenfalls zum Quantifizieren eines Analyten, der gegebenenfalls in einer flüssigen Probe vorhanden ist, umfassend die folgenden Schritte:
i) Inkontaktbringen der flüssigen Probe mit der Oberfläche eines festen Trägers, der mindestens einen aktiven Bereich umfasst, auf dem mindestens eine Sonde, die den Analyten binden kann, immobilisiert ist;
ii) Inkontaktbringen der Oberfläche mit einer Lösung, die mindestens ein Oligonukleotid mit doppelter Stamm-Schleifen-Struktur enthält, das in der Lage ist, an den Analyten zu binden, wobei das Oligonukleotid vor dem Inkontaktbringen synthetisiert worden ist;
iii) Waschen der Oberfläche;
iv) Inkontaktbringen der Oberfläche mit zwei schleifenvermittelten isothermen Amplifikationsprimern unter Bedingungen, die eine Amplifikation des Oligonukleotids ermöglichen;
v) Nachweisen und gegebenenfalls Quantifizieren des Produkts der Amplifikation des Oligonukleotids.

2. Verfahren nach Anspruch 1, **dadurch gekennzeichnet, dass** das Oligonukleotid mit doppelter Stamm-Schleifen-Struktur der folgenden Formel (I) oder (II) entspricht:
5'-F1c-F2-F0'-F1-Int-B1c-B0'-B2c-B1-3' (I)
5'-F1c-F2-F0'-F1-B1c-B0'-B2c-B1-3' (II)
in denen
der F1c-Abschnitt eine Nukleotidsequenz aufweist, die komplementär zur Nukleotidsequenz des F1-Abschnitts ist, wodurch der F1c-Abschnitt und der F1-Abschnitt hybridisieren, um den Stamm der ersten Stamm-Schleifen-Struktur zu bilden,
der F2-F0'-Abschnitt die Schleife der ersten Stamm-Schleifen-Struktur bildet,
der B1-Abschnitt eine Nukleotidsequenz aufweist, die komplementär zur Nukleotidsequenz des B1c-Abschnitts ist, wodurch der B1-Abschnitt und der B1c-Abschnitt hybridisieren, um den Stamm der zweiten Stamm-Schleifen-Struktur zu bilden,
der B0'-B2c-Abschnitt die Schleife der zweiten Stamm-Schleifen-Struktur bildet, und
der Int-Abschnitt den F1-Abschnitt und den B1c-Abschnitt trennt und mindestens ein Nukleotid umfasst.

3. Verfahren nach Anspruch 2, **dadurch gekennzeichnet, dass** in dem Oligonukleotid mit doppelter Stamm-Schleifen-Struktur der Formel (I) oder (II)
der F1-Abschnitt und der F1c-Abschnitt jeweils 10 bis 35 Nukleotide umfassen;
der B1-Abschnitt und der B1c-Abschnitt jeweils 10 bis 35 Nukleotide umfassen;
der F2-Abschnitt 8 bis 25 Nukleotide umfasst und der F0'-Abschnitt so beschaffen ist, dass die Schleife der ersten Stamm-Schleifen-Struktur 10 bis 70 Nukleotide umfasst; und/oder
der B2c-Abschnitt 8 bis 35 Nukleotide umfasst und der B0'-Abschnitt so beschaffen ist, dass die Schleife der zweiten Stamm-Schleifen-Struktur 10 bis 70 Nukleotide umfasst.

4. Verfahren nach Anspruch 2 oder 3, **dadurch gekennzeichnet, dass** in dem Oligonukleotid mit doppelter Stamm-Schleifen-Struktur der Formel (I) oder (II)
der F1-Abschnitt und der F1c-Abschnitt jeweils 21 Nukleotide umfassen; der B1-Abschnitt und der B1c-Abschnitt jeweils 25 Nukleotide umfassen; der F2-Abschnitt 18 Nukleotide umfasst; und/oder
der B2c-Abschnitt 18 Nukleotide umfasst.

5. Verfahren nach einem der Ansprüche 2 bis 4, **dadurch gekennzeichnet, dass** das Oligonukleotid mit doppelter Stamm-Schleifen-Struktur mindestens eine spezifische Sequenz des nachzuweisenden Analyten oder mindestens eine Sequenz, die komplementär zu einem Teil der Nukleotidsequenz des nachzuweisenden Analyten ist, aufweist, wenn Letzterer ein Nukleotidmolekül ist.

6. Verfahren nach Anspruch 5, **dadurch gekennzeichnet, dass** die spezifische Sequenz des nachzuweisenden Analyten oder die Sequenz, die komplementär zu einem Teil der Nukleotidsequenz des Analyten ist, sich im F1-Abschnitt, im F1c-Abschnitt, im B1-Abschnitt, im B1c-Abschnitt, im F0'-Abschnitt, im F2-Abschnitt, im Int-Abschnitt, im B2c-Abschnitt, im B0'-Abschnitt, gleichzeitig im F1c-Abschnitt und im F2-Abschnitt, gleichzeitig im F2-Abschnitt und im F0'-Abschnitt, gleichzeitig im F0'-Abschnitt und im F1-Abschnitt, gleichzeitig im F1c-Abschnitt und im Int-Abschnitt für ein Oligonukleotid der Formel (I), gleichzeitig im F1c-Abschnitt im B1-Abschnitt für ein Oligonukleotid der Formel (II), gleichzeitig im Int-Abschnitt und im B1c-Abschnitt für ein Oligonukleotid der Formel (I), gleichzeitig im B1c-Abschnitt und im B0'-Abschnitt, gleichzeitig im B0'-Abschnitt und im B2c-Abschnitt und gleichzeitig im B2c-Abschnitt und im B1-Abschnitt befindet.

7. Verfahren nach einem der Ansprüche 2 bis 6, **dadurch gekennzeichnet, dass** das Oligonukleotid mit doppelter Stamm-Schleifen-Struktur mindestens eine spezifische Sequenz der Sonde oder eine Sequenz, die komplementär zu einem Teil der Nukleotidsequenz der Sonde ist, aufweist, wenn Letztere ein Nukleotidmolekül ist.

8. Verfahren nach einem der Ansprüche 1 bis 7, **dadurch gekennzeichnet, dass** die beiden im Schritt iv) eingesetzten Primer der folgenden Formel (III) bzw. der folgenden Formel (IV) entsprechen:
5'-F1c-F2-3' (III)
5'-B1c-B2-3' (IV)
wobei der B2-Abschnitt eine Nukleotidsequenz aufweist, die komplementär zur Nukleotidsequenz des B2c-Abschnitts ist,
wobei die Abschnitte F1c, F2, B1c und B2c wie in einem der Ansprüche 2 bis 4 definiert sind.

9. Verfahren nach einem der Ansprüche 1 bis 8, **dadurch gekennzeichnet, dass** das Amplifikationsprodukt während Schritt v) durch eine Messung der Trübung, durch eine pH-Sonde, durch Gelelektrophorese, durch einen Farbtest oder durch eine Fluoreszenzmessung nachgewiesen wird.

10. Verfahren nach einem der Ansprüche 1 bis 9, **dadurch gekennzeichnet, dass** nach Schritt i) und vor Schritt ii) die Oberfläche des festen Trägers gewaschen wird.

## Claims

1. Method for detecting and optionally quantifying an analyte possibly present in a liquid sample, comprising the following steps:
i) contacting said liquid sample with the surface of a solid support comprising at least one active zone on which at least one probe capable of binding said analyte is immobilised;
ii) contacting said surface with a solution containing at least one oligonucleotide with a double stem-loop structure, capable of binding to said analyte, said oligonucleotide having been synthesised prior to said contacting;
iii) washing said surface;
iv) contacting said surface with two loop-mediated isothermal amplification primers in conditions permitting the amplification of said oligonucleotide;
v) detecting and optionally quantifying the amplification product of said oligonucleotide.

2. Method according to claim 1, **characterised in that** said oligonucleotide with a double stem-loop structure corresponds to the formula (I) or (II) below:
5'-F1c-F2-F0'-F1-Int-B1c-B0'-B2c-B1-3' (I)
5'-F1c-F2-F0'-F1-B1c-B0'-B2c-B1-3' (II)
in which
the F1c portion has a nucleotide sequence that is complementary to the nucleotide sequence of portion F1 whereby portion F1c and portion F1 hybridise to form the stem of the first stem-loop structure,
the portion F2-F0' forms the loop of the first stem-loop structure,
the portion B1 has nucleotide sequence that is complementary to the nucleotide sequence of portion B1c whereby portion B1 and portion B1c hybridise to form the stem of the second stem-loop structure,
the portion B0'-B2c forms the loop of the second stem-loop structure, and
the portion Int separates portion F1 and portion B1c and comprises at least one nucleotide.

3. Method according to claim 2, **characterized in that** in said oligonucleotide with a double stem-loop structure of formula (I) or (II),
said portion F1 and said portion F1c each comprise 10 to 35 nucleotides;
said portion B1 and said portion B1c each comprise 10 to 35 nucleotides;
said portion F2 comprises 8 to 25 nucleotides and said portion F0' is such that the loop of the first stem-loop structure comprises 10 to 70 nucleotides; and/or
said portion B2c comprises 8 to 35 nucleotides and said portion B0' is such that the loop of the second stem-loop structure comprises 10 to 70 nucleotides.

4. Method according to claim 2 or 3, **characterized in that** in said oligonucleotide with a double stem-loop structure of formula (I) or (II),
said portion F1 and said portion F1c each comprise 21 nucleotides;
said portion B1 and said portion B1c each comprise 25 nucleotides;
said portion F2 comprises 18 nucleotides; and/or
said portion B2c comprises 18 nucleotides.

5. Method according to any one of claims 2 to 4, **characterised in that** said oligonucleotide with a double stem-loop structure has at least one sequence specific to the analyte to be detected or at least one sequence complementary to a part of the nucleotide sequence of the analyte to be detected when the latter is a nucleotide molecule.

6. Method according to claim 5, **characterised in that** said specific sequence of the analyte to be detected or said sequence complementary to a part of the nucleotide sequence of the analyte is found in portion F1, in portion F1c, in portion B1, in portion B1c, in portion F0', in portion F2, in portion Int, in portion B2c, in portion B0', in both portion F1c and portion F2, in both portion F2 and portion F0', in both portion F0' and portion F1, in both portion F1c and portion Int for an oligonucleotide of formula (I), both in portion F1c and portion B1 for an oligonucleotide of formula (II), both in portion Int and portion B1c for an oligonucleotide of formula (I), both in portion B1c and portion B0', both in portion B0' and portion B2c, both in portion B2c and portion B1.

7. Method according to any one of claims 2 to 6, **characterised in that** said oligonucleotide with a double stem-loop structure has at least one sequence specific to said probe or a sequence complementary to a part of the nucleotide sequence of the probe when the latter is a nucleotide molecule.

8. Method according to any one of claims 1 to 7, **characterized in that** the two primers used in said step iv) correspond respectively to formula (III) and formula (IV) below:
5'-F1c-F2-3' (III)
5'-B1c-B2-3' (IV)
the portion B2 has a nucleotide sequence complementary to the nucleotide sequence of portion B2c,
portions F1c, F2, B1c and B2c being as defined in any one of claims 2 to 4.

9. Method according to any one of claims 1 to 8, **characterized in that** said amplification product is detected, during said step v), by measuring the turbidity, by a pH probe, via a gel electrophoresis, by a colorimetric test or by measuring fluorescence.

10. Method according to any one of claims 1 to 9, **characterized in that** following step i) and prior to said step ii), the surface of the solid support is washed.
